# EUROPEAN PATENT SPECIFICATION

(11) **EP 3 435 963 B1**
(45) Date of publication and mention of the grant of the patent: **01.05.2024**
(21) Application number: 17713638.9
(22) Date of filing: 24.03.2017
(51) Int. Cl.: A61K 8/34, A61Q 1/04, A61K 8/81, A61K 8/891, A61K 8/06, A45D 34/04, A45D 40/26

(54) **EMULSION COMPRISING A FILM-FORMING AGENT AND NON-VOLATILE OILS AND COSMETIC PROCESS**
EMULSION MIT EINEM FILMBILDENDEN MITTEL UND NICHTFLÜCHTIGEN ÖLEN UND KOSMETISCHES VERFAHREN
EMULSION COMPRENANT UN AGENT FILMOGÈNE ET DES HUILES NON VOLATILES ET PROCÉDÉ COSMÉTIQUE

(30) Priority: 31.03.2016 FR 1652850
(43) Date of publication of application: 06.02.2019
(73) Proprietor: L'OREAL, 75008 Paris (FR)
(72) Inventor: CAVAZZUTI, Roberto, 94152 CHEVILLY LA RUE (FR); LAHOUSSE, Florence, 94152 CHEVILLY LA RUE (FR); HENIN, Emilie, 94152 CHEVILLY LA RUE (FR); ARDITTY, Stéphane, 94152 CHEVILLY LA RUE (FR); PRUD'HOMME, Estelle, 94152 CHEVILLY LA RUE (FR); GUILLARD, Sylvie, 94152 CHEVILLY LA RUE (FR); MONDON, Virginie, 94152 CHEVILLY LA RUE (FR); SANCHEZ, Marcel, 92110 Clichy (FR)
(74) Representative: L'Oreal
(86) International application number: PCT/EP2017/057123
(87) International publication number: WO 2017/167668

(56) References cited:
- EP-A1- 0 612 488
- EP-A1- 0 688 516
- EP-A1- 0 756 864
- EP-A2- 1 293 448
- FR-A1- 3 015 251
- US-A1- 2012 263 662
- US-B1- 6 305 863
- STÉPHANIE POSTIAUX KATRIN STEINBACH ESTERINA SCHIROSI ET AL: "Long lasting and meal resistance in lipsticks and lipcreams using silicone acrylate copolymers", RESEARCH DISCLOSURE, MASON PUBLICATIONS, HAMPSHIRE, GB, vol. 505, no. 25, 1 May 2006 (2006-05-01), XP007136165, ISSN: 0374-4353

## Description

The subject of the present invention is a cosmetic composition in the form of a liquid emulsion comprising a particular film-forming polymer, and also a process for making up and/or caring for the lips in particular using the device and the composition.

The present invention relates to the field of making up and/or caring for the lips using fluid compositions.

The development of fluid compositions dedicated to making up and/or caring for the lips, such as liquid lipsticks, which are stable and endowed with satisfactory properties in terms of application (guidance on application, ease of spreading and fineness of the deposit), but also in terms of the makeup effect of the deposit on the lips, for instance the coverage and the absence of migration of the deposit, preferably without becoming tacky, is an ongoing objective.

Generally, formulations corresponding to anhydrous liquid galenical formulations conventionally comprise oils, which in particular provide gloss, optionally waxes for structuring the compositions, fillers, in particular for thickening the composition, film-forming polymers, and colorants.

In the more particular case of compositions providing coverage, it is important for the latter to be easy to apply to the lips, precisely and as an even layer. In addition, the deposit is not expected to migrate, which would result in the outline of the lips being made imprecise.

With the conventional lipstick compositions of this type, generally used with dipping applicators which have a flocked end piece, it is noted that the deposit is relatively thick, thereby giving it a more or less tacky nature, in particular induced by the use of these oils and of the polymers present. This nature is in particular reflected by a phenomenon of the made up lips sticking to one another, which is therefore unpleasant in terms of comfort for the user.

Another difficulty encountered with liquid lipsticks lies in the fact that the composition must be sufficiently fluid to be easily applied, but not too fluid, so as to avoid losing stability of the composition (pigment sedimentation) and losing ease of application (running and/or migration of the composition to the wrinkles and fine lines of the area around the lips).

Patent application FR-A-3015251 discloses a liquid water-in-oil emulsion containing a dendrimer film-forming polymer, an aqueous phase and a liquid fatty phase which may contain volatile and/or non-volatile oils.

Compositions which at the same time provide very good coverage of the lips, as a precise deposit, which does not migrate, and for which the tacky nature has been virtually dispensed with, and a device which would make it possible to apply them, are therefore sought.

These objectives are achieved by means of the present invention, a subject of which is a cosmetic composition which is in the form of a liquid emulsion comprising:
∘ water;
∘ at least one film-forming agent chosen from vinyl polymers comprising at least one carbosiloxane dendrimer-based unit; chosen from polymers of which the INCI name is Acrylates / Polytrimethylsiloxy Methacrylate Copolymer;
∘ from 5% to 15% by weight, relative to the weight of the composition, of at least one polar non-volatile hydrocarbon-based oil;
∘ from 1% to 8% by weight, relative to the weight of the composition, of at least one non-volatile phenyl silicone oil, preferably without dimethicone fragment.

Furthermore, the invention relates to a process for making up and/or caring for the lips, in which the abovementioned composition is applied to the lips, in particular, by means of the abovementioned device.

Application devices that are particularly suited to this composition will be described with reference to the appended drawings, in which:
- Figure 1 is a diagrammatic representation in longitudinal section of a packaging and applying assembly.
- Figure 2 is a diagrammatic representation in longitudinal section of an applicator member equipping the assembly of Figure 1.
- Figure 3 is a diagrammatic representation in longitudinal section of a ring forming a housing for the applicator member of Figure 2.
- Figure 4 is a diagrammatic representation viewed from above of the ring of Figure 3.

The film deposited on the lips has the advantage of being very thin and of being virtually imperceptible to the user. It is thus a very light deposit that is deposited on the lips by means of the device . This deposit is also non-tacky, and does not introduce a sensation of dryness. The deposit thus obtained does not migrate, does not transfer, and has a very good wear property.

It should be noted that, in the remainder of the description, unless otherwise indicated, the limits indicated for a range are included in that range.

The contents are indicated relative to the weight of the composition, in other words, relative to the weight of the total composition.

The expressions "at least one" and "several" are used without distinction.

### DEVICE

This device is in particular described in French Patent applications n° 1559092 filed on 25/09/15 and n° 1651670 filed on 29/02/16.

More particularly, the device comprises, on the one hand, a body forming a reservoir intended to contain the cosmetic composition to be applied and, on the other hand, an applicator comprising an applicator member defining a convex application surface having at least one apex.

The body forming a reservoir comprises a housing capable of receiving the applicator member and of which one dividing wall with the reservoir has at least one through-orifice in direct fluid communication with the reservoir. Said orifices are located only at at least one base of the housing opposite the vicinity of an apex of the applicator member when said applicator member is in place in the housing of the body forming a reservoir.

The packaging and applying assembly is characterized in that the applicator member is made from an open-cell porous material.

The presence of an applicator member defining a convex application surface having an apex or a tip allows in particular a precise application of the composition.

The orifices made in the wall of the housing for receiving the applicator member make it possible in particular to load the applicator member with composition, in particular when the reservoir is shaken or turned upside down.

The expression "orifice located only at at least one base of the housing" is intended to mean that the remainder of the dividing wall is solid, that is to say not pierced.

Thus, when combining one or more distribution orifices in direct fluid communication, arranged in a very localized manner with a porous application surface, it has been noted, surprisingly, that the packaging and applying assembly targeted allows a precise, comfortable and uniform application of the cosmetic product contained in the reservoir.

The use of very localized orifices also makes it possible to limit the passing of the composition through the dividing wall and thus to obtain a precise and sufficient dose of composition, without overloading the applicator, consequently making it possible to obtain a thin deposit.

The remainder of the application surface, in particular a peripheral or side zone, can be used to tone down the composition applied and to refine the make-up.

The term "direct fluid communication" is intended to mean that the orifice is not linked to a distributing means with a selected opening of the pump or aerosol type making it possible to force the cosmetic composition to pass through said orifice. The loading of the applicator member is done by simply shaking the reservoir or turning it upside down.

This does not prevent the use of a selective closure mechanism making it possible in particular to ensure that the reservoir is leak tight when it is not used, the passage of the composition through the orifice remaining unforced.

In the present application, the expression "vicinity of an apex of the applicator member" targets an apex portion extending in the vicinity of the isolated geometric apex. It may be considered that the orifices are located in a zone around the geometric apex representing at most 5% or even 10 % of the total surface area of the application surface.

Preferentially, the orifices are located at a base of the housing corresponding to an absolute apex of the applicator member, preferably only at said base.

According to a first implementation variant, the wall of the housing has a single orifice.

According to a second implementation variant, the wall of the housing has a plurality of orifices.

The presence of a plurality of orifices makes it possible to retain orifices of relatively small size, making it possible to ensure the passage of the most homogeneous possible amount of composition while at the same time ensuring a total passage surface area is sufficient for good loading of the applicator member, after for example only a few shaking movements. In addition, the size of the orifices is also determined according to the size of the solid particles so as to prevent or limit blocking phenomena.

In the case of several orifices, the latter are preferentially distributed uniformly around the centre of the base. This makes it possible to ensure optimal loading of the application tip.

Advantageously, additionally, the wall of the housing has a central orifice, located at the centre of the base. The term "centre of the base" is intended to mean the tip opposite the geometric apex of the applicator.

According to one preferred embodiment, all or some of the orifices have a substantially circular cross section. Such a circular-shaped cross section is very suitable for preventing blocking phenomena. Where appropriate, depending on the particles used, other cross sections can be envisaged.

Advantageously additionally, the reservoir comprises at least one mixing element, in particular at least one mixing bead. The characteristics of the mixing element, such as the weight or the size, will be chosen according to the properties of the cosmetic composition.

The applicator member can preferably be made of a foam, it is preferentially a block of foam.

Preferentially, the applicator member has an application surface that is at least partially flocked, preferably totally flocked.

According to one variant which is not represented, the support and fixing system comprises at least one complementary clamping ring capable of compressing a part of the mounting foot located at a non-zero distance from a base surface of said mounting foot oriented towards the base wall of the support and fixing system when the applicator member is placed in the housing of the support and fixing system, the clamping ring having at least one clip-fastening means capable of cooperating with at least one complementary clip-fastening means of the peripheral skirt of the support and fixing system.

The clamping ring can be made dismountable so as to optionally allow the applicator member to be replaced with an identical, for example new, or different applicator member.

Moreover, said clamping ring compresses only one part of the mounting foot of the applicator member at a distance from a base of said mounting foot. It follows that the mounting foot has a compressed portion, of reduced cross section, and a base portion which is non-compressed or less compressed, having a greater cross section. The non-compressed or less compressed base portion has a wider cross section which opposes any extraction movement in a longitudinal component, including side travel movements, of the applicator member outside the ring and greatly contributes to the fixing thereof and the holding in place thereof. In addition, the compression of a part of the mounting foot of the applicator member increases the friction between the mounting foot and the ring, thereby also contributing to the overall holding in place.

Advantageously, the degree of compression is at least 10%, preferably at least 20%, or even at least 30%, relative to the cross section of the applicator member in the non-compressed or less compressed state in the housing, and preferably less than 40%, or even 35%.

Additionally advantageously, the base wall of the support and fixing system comprises at least one notch capable of penetrating into the base surface of the foot of the applicator member. The notches contribute to the fixing of the applicator member by limiting side movements, in particular rotation movements.

Additionally advantageously, said clamping ring has an end, preferentially intended to be oriented towards the base wall of the support and fixing system, said end having an at least partially peripheral radial shoulder capable of cooperating with a complementary at least partially peripheral groove of the mounting foot of the applicator member.

Preferentially, the peripheral radial shoulder has a surface intended to be oriented towards the base wall of the support and fixing system and which has at least one attaching notch capable of penetrating into a corresponding surface of the peripheral groove of the applicator member.

According to one preferred embodiment the groove of the mounting foot of the applicator member is located substantially half way up said mounting foot, more particularly between 30% and 60% of the height of said mounting foot relative to its base.

Additionally, the base wall of the support and fixing system has at least one pin, not represented, which is in particular central, extending substantially perpendicular to the base wall at least partially through the clamping ring and capable of penetrating into a corresponding cavity of the mounting foot of the applicator member.

Preferentially, the pin extends over the entire length of the clamping ring and totally passes through it.

Preferentially, the pin of the base wall juts out from the skirt of the support and fixing system. Such a characteristic helps to limit the amplitude of travel of the applicator member.

Advantageously, the clamping ring extends over approximately half the height of the mounting foot of the applicator member. Preferentially, the clamping ring extends over at least 40% of the height of the mounting foot and over at most 90% of the height of the mounting foot.

Advantageously, the applicator is configured so as to fix onto the container when not in use, the applicator member being received in the housing. Thus, the applicator makes it possible to close the reservoir.

Preferentially, the application surface exhibits no surface in particular no concavity, capable of forming a space with the wall of the housing when the applicator member is received in the housing. In particular, the application surface exhibits no concavity in comparison with the orifices. This is because the presence of a concavity could be capable of causing the formation of a cavity between the applicator and the dividing wall of the housing, inside of which cavity product could accumulate, which is not desirable.

Preferably, the applicator member has a conical general shape, in particular with a rounded tip, or hemispherical general shape. Such a shape is particularly suitable for an application to the lips.

More preferably, the orifices are blocked by the application surface when the applicator is fixed onto the container. This makes it possible to prevent any leaking of the cosmetic composition through the orifices when the applicator is in the closed position.

Additionally advantageously, the application surface at least partially deforms against the wall of the housing when the applicator is fixed onto the container, the applicator member being slightly compressed. Thus, it is possible, depending on the characteristics of the cosmetic composition, to place the applicator member under slight compression in order to add a pump effect making it possible to facilitate the diffusion of the composition. This compression also improves the blocking of the orifices by the applicator member.

The present invention will be understood more clearly on reading the detailed description which follows, from the viewpoint of the appended drawings in which:

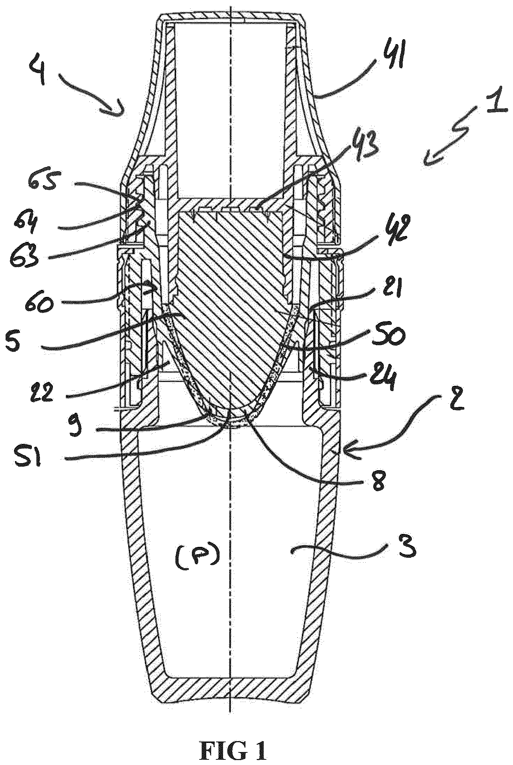

- Figure 1 is a diagrammatic representation in longitudinal section of a packaging and applying assembly.

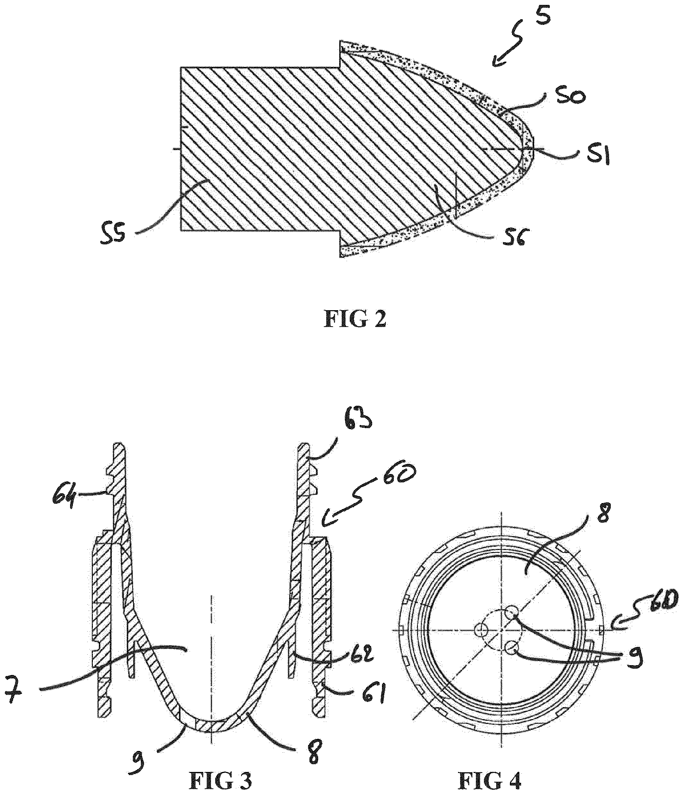

- Figure 2 is a diagrammatic representation in longitudinal section of an applicator member equipping the assembly of Figure 1.
- Figure 3 is a diagrammatic representation in longitudinal section of a ring forming a housing for the applicator member of Figure 2.
- Figure 4 is a diagrammatic representation viewed from above of the ring in Figure 3.

Figure 1 shows an assembly 1 for packaging and applying the cosmetic composition P.

The assembly 1 comprises a body 2 forming a reservoir 3 containing the cosmetic composition P to be applied and an applicator 4 comprising an applicator member 5 defining a convex application surface 50 having at least one apex 51.

The body 2 has a free upper edge 21 delimiting an opening 22 of said reservoir 3. More specifically, the opening 22 is located at a free upper end of a collar 24 of the body 2.

The body 2 has a symmetry of rotation. The opening 22 has a circular cross section. Of course, other shapes can be envisaged.

The body 2 has, at the opening 22, a housing 7 capable of receiving the applicator member 5, said housing 7 being at least partially delimited by a dividing wall 8 of the reservoir 3.

In accordance with the present application, the dividing wall 8 has at least one through-orifice 9 in direct fluid communication with the reservoir 3.

The dividing wall 8 can be attached to the reservoir 3, as illustrated, or can be made of a single part by moulding the body of the reservoir.

In the case in point, the dividing wall 8 is borne by a ring 60 surmounting the opening 22.

The ring 60 may be mounted on the body 2 by any means, in particular by clip-fastening or adhesive bonding. It may be removable or non-removable.

In the case in point, the ring 60 is capable of coming into contact with the collar 24.

To do this, the ring 60 comprises an external peripheral wall 61 and an internal peripheral wall 62 together defining a mounting skirt.

The mounting skirt also ensures leak tightness with respect to the product.

Alternatively, a ring as represented in Figure 10J of document FR 296 2890 can also be envisaged.

The applicator 4 exceptionally comprises a grasping part 41 on which the applicator member 5 is mounted.

The grasping part 41 forms a cap and is capable of coming into contact, in a removable manner, with an upper peripheral wall 63 of the ring 6, thus sealing the reservoir 3 closed. More particularly, the peripheral wall 63 of the ring 6 bears an external male screw thread 64 capable of cooperating with a complementary internal tapped thread 65 of the grasping part.

When not in use, and when the grasping part 41 is screwed onto the ring 6, the applicator member 5 is received in the housing 7 (Figure 1).

In accordance with the present application, the applicator member 5 is made of an open-porosity porous material, for example of a foam.

In particular, the applicator member 5 is made of a polyurethane foam, in particular of S90 type (standard DIN 4102-9).

The applicator member 5 is mounted on the grasping part 41 by any known means, in particular by assembly, clip-fastening or snap-fastening.

In the case in point, the applicator member 5 has a substantially cylindrical foot 55 which is introduced into a corresponding pillar 42 of the grasping part. A base wall 43 of the pillar 42 increases the fixing surface. The base wall 43 also provides translational blocking.

Opposite the foot 55, the applicator member 5 has a head 56 defining the application surface 51. The head 56 has a generally conical shape. The head 56 has a rounded free end or point forming the apex 51. In the case in point, the apex 51 is consequently an absolute apex.

The head 56 has a base which has a diameter that is slightly larger than the diameter of the foot 55 and thus has a shoulder 54 capable of bearing against an edge of the pillar 42. This shoulder 57 makes it possible in particular to give the applicator member better strength.

The housing 7 is conformed so as to have a shape substantially complementary to the head 56.

As can be seen in Figure 1, when the applicator 4 is fixed onto the reservoir 3 and the applicator member 5 is in place in the housing, the apex 51 is slightly compressed.

It will also be noted that the application surface 51 is flocked. For the application of the flock, use may be made of an adhesive that is relatively rigid after drying. Thus, drying the adhesive contributes to making the applicator member 5 rigid.

As previously indicated, the housing 7 is in communication with the interior of the reservoir 3 by means of the perforated dividing wall 8, passing through which are orifices 9 that are constantly open.

In accordance with the present application, the orifices 9 are located only at at least one base of the housing in comparison to the vicinity of the apex 51 of the applicator member 5 when said applicator member 5 is placed in the housing 7 of the body 2 forming a reservoir 3.

In the case in point, the wall 8 comprises three orifices 9. The orifices 9 are regularly distributed in proximity to and around the centre of the base of the wall 8. The orifices have substantially circular cross section. In the case in point, the diameter of the orifices is 1.5 mm.

The total surface area of the orifice(s) is thus approximately 5.3 mm². Preferably, the total surface area of the openings is less than 5.5 mm². The total surface area of each opening is also sufficient to limit or even prevent blocking phenomena.

The centre of each orifice 9 is located at a distance of 4.5 mm from the centre of the base corresponding to the apex 51 of the applicator member. Preferably, the orifices are located at less than 5 mm from the centre of the base.

The applicator member 5 advantageously blocks all the orifices of the wall 8 when the applicator 4 is in place on the reservoir 3. This can be useful for preventing any excess composition from spilling in the housing when the applicator closes said housing.

Advantageously, the reservoir 3 comprises at least one mixing element, in particular at least one mixing bead (not represented).

The packaging and applying assembly 1 is used in the following way.

Since the assembly 1 is in closed configuration, the user can shake the assembly or turn it upside down.

In doing so, product is projected through the orifices 9 of the dividing wall 8 and very locally impregnates the applicator member 5 at an apex 51 of its application surface.

After a few shaking movements, the user unscrews the applicator 4 and grasps said applicator 4 by the grasping part 41 and disconnects it from the reservoir 3. In doing so, the applicator member 5 is extracted from the housing 7 and the application surface is accessible to the user who can then carry out the application.

To do this, the user brings the apex portion of the applicator member into contact with the application surface to be covered, for example a surface of the lips, so as to deposit thereon the cosmetic composition.

Where appropriate, said user can use a side portion of the application surface to tone down or spread the composition.

### COMPOSITION

As indicated previously, the composition, particularly used in the device that has just been described, is in the form of a liquid emulsion.

The term "liquid" is intended to mean a fluid texture, the viscosity of which at 25°C is more particularly between 0.005 and 12 Pa.s, preferably between 0.01 and 10 Pa.s and even more advantageously between 0.05 and 8 Pa.s.

Preferably, the viscosity at 25°C of a composition according to the invention is between 0.1 and 6 Pa.s.

### Protocol for measuring the viscosity:

The viscosity measurement is generally performed at 25°C, using a Rheomat RM180 viscometer equipped with a No. 2 or 3 spindle, the measurement being performed after 10 minutes of rotation of the spindle in the formula, at a shear rate of 200 revolutions/min (rpm).

The composition may be in the form of a direct (oil-in-water) or inverse (water-in-oil) emulsion.

According to one preferred embodiment of the invention, the composition is in the form of an inverse (water-in-oil) emulsion.

Moreover, the composition according to the invention comprises, in addition to the water, at least one particular film-forming polymer and at least non-volatile oils, one of which is chosen from polar hydrocarbon-based non-volatile oils, the other of which is chosen from phenyl silicone non-volatile oils, preferably free of dimethicone fragment.

The composition according to the invention could also comprise other additional non-polar hydrocarbon-based or non-phenyl silcone non-volatile oils.

The total content of non-volatile oils preferably represents from 6 to 20% by weight, and in accordance with an even more particular embodiment, from 6 to 15% by weight, relative to the weight of the composition.

### POLAR NON-VOLATILE HYDROCARBON-BASED OILS

As previously indicated, the composition used in the device in accordance with the invention comprises at least one polar non-volatile hydrocarbon-based oil.

The term "oil" is intended to mean a water-immiscible non-aqueous compound which is liquid at 25°C and atmospheric pressure (1.013 × 10⁵ Pa).

The term "immiscible" is intended to mean that the mixing of the same amount of water and oil, after stirring, does not result in a stable solution comprising only a single phase, under the above-mentioned temperature and pressure conditions. Observation is carried out by eye or using a phase contrast microscope, if necessary, on 100 g of mixture obtained after sufficient Rayneri stirring to produce a vortex within the mixture (by way of indication, 200 to 1000 rev/min), the resulting mixture being left standing, in a closed flask, for 24 hours at ambient temperature before observation.

The term "non-volatile oil" is intended to mean an oil of which the vapour pressure at 25°C and atmospheric pressure is non-zero and is less than 10⁻³ mmHg (0.13 Pa).

The term "hydrocarbon-based oil" is intended to mean an oil formed essentially from, or even constituted of, carbon and hydrogen atoms, and optionally oxygen and nitrogen atoms, and not containing any silicon or fluorine atoms.

It can contain alcohol, ester, ether, carboxylic acid, amine and/or amide groups.

Preferably, the hydrocarbon-based oil is, in addition to being free of silicon and fluorine, free of heteroatoms such as nitrogen, sulfur and phosphorus. The hydrocarbon-based oil is thus distinct from a silicone oil and a fluorinated oil.

In the present case, the polar non-volatile hydrocarbon-based oil comprises at least one oxygen atom.

In particular, this non-volatile hydrocarbon-based oil comprises at least one alcohol function (it is then an "alcohol oil") and/or at least one ester function (it is then an "ester oil").

The ester oils that may be used in the compositions according to the invention may in particular be hydroxylated.

The composition according to the invention may comprise one or more non-volatile hydrocarbon-based oils, in particular chosen from:
- C₁₀-C₂₆ alcohols, preferably monoalcohols;

More particularly, the C₁₀-C₂₆ alcohols are saturated or unsaturated and branched or unbranched and comprise from 10 to 26 carbon atoms.

Preferably, the C₁₀-C₂₆ alcohols are fatty alcohols, which are preferably branched when they comprise at least 16 carbon atoms.

As examples of fatty alcohols that may be used according to the invention, mention may be made of linear or branched fatty alcohols, of synthetic origin or alternatively of natural origin, for instance alcohols derived from plant materials (coconut, palm kernel, palm, etc.) or animal materials (tallow, etc.).

Needless to say, other long-chain alcohols may also be used, for instance ether alcohols or alternatively "Guerbet" alcohols.

Finally, use may also be made of certain fractions of alcohols of varying length of natural origin, for instance coconut (C₁₂ to C₁₆) or tallow (C₁₆ to C₁₈) or compounds of diol or cholesterol type.

Preferably, a fatty alcohol comprising from 10 to 24 carbon atoms is used.

Mention may in particular be made, as specific examples of fatty alcohols which can preferably be used, of lauryl alcohol, isostearyl alcohol, oleyl alcohol, 2-butyloctanol, 2-undecylpentadecanol, 2-hexyldecyl alcohol, isocetyl alcohol, octyldodecanol and mixtures thereof.
- optionally hydroxylated monoesters, diesters or triesters of a C₂-C₈ monocarboxylic or polycarboxylic acid and of a C₂-C₈ alcohol.

In particular:
* optionally hydroxylated monoesters of a C₂-C₈ carboxylic acid and of a C₂-C₈ alcohol,
* optionally hydroxylated diesters of a C₂-C₈ dicarboxylic acid and of a C₂-C₈ alcohol, such as diisopropyl adipate, 2-diethylhexyl adipate, dibutyl adipate, diisostearyl adipate or 2-diethylhexyl succinate,
* optionally hydroxylated triesters of a C₂-C₈ tricarboxylic acid and of a C₂-C₈ alcohol, such as citric acid esters, such as trioctyl citrate, triethyl citrate, acetyl tributyl citrate, tributyl citrate or acetyl tributyl citrate;

- esters of a C₂-C₈ polyol and of one or more C₂-C₈ carboxylic acids, such as glycol diesters of monoacids, such as neopentyl glycol diheptanoate, or glycol triesters of monoacids, such as triacetin.
- ester oils, in particular having between 18 and 70 carbon atoms.

Examples that may be mentioned include monoesters, diesters or triesters.

The ester oils may be hydroxylated or non-hydroxylated.

The non-volatile ester oil may for example be chosen from:
* monoesters comprising between 18 and 40 carbon atoms in total, in particular the monoesters of formula R₁COOR₂ in which R₁ represents a saturated or unsaturated, linear or branched or aromatic fatty acid residue comprising from 4 to 40 carbon atoms and R₂ represents a hydrocarbon-based chain, which is in particular branched, containing from 4 to 40 carbon atoms, on condition that R₁ + R₂ ≥ 18, for instance Purcellin oil (cetostearyl octanoate), isononyl isononanoate, C₁₂ to C₁₅ alkyl benzoate, 2-ethylhexyl palmitate, octyldodecyl neopentanoate, 2-octyldodecyl stearate, 2-octyldodecyl erucate, isostearyl isostearate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate or 2-octyldodecyl myristate.

Preferably, they are esters of formula R₁COOR₂ in which R₁ represents a linear or branched fatty acid residue containing from 4 to 40 carbon atoms and R₂ represents a hydrocarbon-based chain that is in particular branched, containing from 4 to 40 carbon atoms, R₁ and R₂ being such that R₁ + R₂ ≥ 18.

Even more particularly, the ester comprises between 18 and 40 carbon atoms in total.

Preferred monoesters that may be mentioned include isononyl isononanoate, oleyl erucate and/or 2-octyldodecyl neopentanoate;
* monoesters of a fatty acid, in particular of 18 to 22 carbon atoms, and in particular of lanolic acid, oleic acid, lauric acid or stearic acid, and of diols, for instance propylene glycol monoisostearate;
* diesters, in particular comprising between 18 and 60 carbon atoms in total and in particular between 18 and 50 carbon atoms in total. Use may be made especially of diesters of a dicarboxylic acid and of monoalcohols, preferably such as diisostearyl malate, or glycol diesters of monocarboxylic acids, such as neopentyl glycol diheptanoate, propylene glycol dioctanoate, diethylene glycol diisononanoate or polyglyceryl-2 diisostearate (in particular such as the compound sold under the commercial reference Dermol DGDIS by the company Alzo);
* hydroxylated monoesters and diesters, preferably with a total carbon number ranging from 18 to 70, for instance polyglyceryl-3 diisostearate, isostearyl lactate, octyl hydroxystearate, octyldodecyl hydroxystearate, diisostearyl malate or glyceryl stearate;
* triesters, in particular comprising between 35 and 70 carbon atoms in total, in particular such as triesters of a tricarboxylic acid, such as triisostearyl citrate, or tridecyl trimellitate, or glycol triesters of monocarboxylic acids such as polyglyceryl-2 triisostearate;
* tetraesters, in particular with a total carbon number ranging from 35 to 70, such as pentaerythritol or polyglycerol tetraesters of a monocarboxylic acid, for instance pentaerythrityl tetrapelargonate, pentaerythrityl tetraisostearate, pentaerythrityl tetraisononanoate, glyceryl tris(2-decyl)tetradecanoate, polyglyceryl-2 tetraisostearate or pentaerythrityl tetrakis(2-decyl)tetradecanoate;
* polyesters obtained by condensation of an unsaturated fatty acid dimer and/or trimer and of diol, such as those described in patent application FR 0 853 634, in particular such as dilinoleic acid and 1,4-butanediol. Mention may in particular be made on this account of the polymer sold by Biosynthis under the name Viscoplast 14436H (INCI name: dilinoleic acid/butanediol copolymer) or else copolymers of polyols and of diacid dimers, and their esters, such as Hailucent ISDA;
* esters and polyesters of diol dimer and of monocarboxylic or dicarboxylic acid, such as esters of diol dimer and of fatty acid and esters of diol dimer and of dicarboxylic acid dimer, in particular which may be obtained from a dicarboxylic acid dimer derived in particular from the dimerization of an unsaturated fatty acid especially of C₈ to C₃₄, especially of C₁₂ to C₂₂, in particular of C₁₆ to C₂₀ and more particularly of C₁₈, such as esters of dilinoleic diacids and of dilinoleic diol dimers, for instance those sold by the company Nippon Fine Chemical under the trade names Lusplan DD-DA5^{®} and DD-DA7^{®};
* polyesters resulting from the esterification of at least one triglyceride of hydroxylated carboxylic acid(s) with an aliphatic monocarboxylic acid and with an aliphatic dicarboxylic acid, which is optionally unsaturated, for instance the succinic acid and isostearic acid castor oil sold under the reference Zenigloss by Zenitech;
* hydrocarbon-based plant oils such as fatty acid triglycerides (which are liquid at ambient temperature), especially of fatty acids containing from 7 to 40 carbon atoms, such as heptanoic or octanoic acid triglycerides or jojoba oil; mention may be made in particular of saturated triglycerides such as caprylic/capric triglyceride and mixtures thereof, for example such as the product sold under the reference Myritol 318 from Cognis, glyceryl triheptanoate, glyceryl trioctanoate, and C₁₈₋₃₆ acid triglycerides such as those sold under the reference Dub TGI 24 by Stéarineries Dubois, and unsaturated triglycerides such as castor oil, olive oil, ximenia oil and pracaxi oil;

- vinylpyrrolidone/1-hexadecene copolymers, for instance the product sold under the name Antaron V-216 (also known as Ganex V216) by the company ISP (MW = 7300 g/mol).
- C₁₂-C₂₆ fatty acids, preferably C₁₂-C₂₂ fatty acids, which are preferably unsaturated, such as oleic acid, linoleic acid or linolenic acid, and mixtures thereof.
- dialkyl carbonates, the 2 alkyl chains possibly being identical or different, such as dicaprylyl carbonate sold under the name Cetiol CC^{®} by Cognis.
- and mixtures thereof.

At least one fatty alcohol comprising from 20 to 26 carbon atoms, and most particularly octyldodecanol is preferably used as polar non-volatile hydrocarbon-based oil.

Preferably, the content of non-volatile polar hydrocarbon-based oil(s) is between 5% and 15% by weight and preferably from 6% to 12% by weight relative to the weight of the composition.

### NON-VOLATILE PHENYL SILICONE OILS

The composition used in the device in accordance with the invention also comprises at least one non-volatile phenyl silicone oil, preferably without dimethicone fragment.

The term "silicone oil" is intended to mean an oil containing at least one silicon atom and in particular containing Si-O groups.

The expression "phenyl silicone oil" denotes a silicone oil bearing at least one phenyl substituent.

It should be noted that the term "dimethicone fragment" denotes a divalent siloxane group in which the silicon atom bears two methyl radicals, this group not being located at the ends of the molecule. It can be represented by the following formula: -(Si(CH₃)₂-O)-.

The silicone oils generally have a molecular weight of less than or equal to 150 000 g/mol, preferably less than or equal to 100 000 g/mol and better still less than or equal to 10 000 g/mol. The weight-average molecular weights are measured in a manner that is conventional in the field, for example using gel permeation chromatography coupled to static light scattering (GPC-MALLS).

The non-volatile phenyl silicone oil may thus be chosen from:
- phenyl silicone oils optionally having a dimethicone fragment corresponding to formula (I) below: 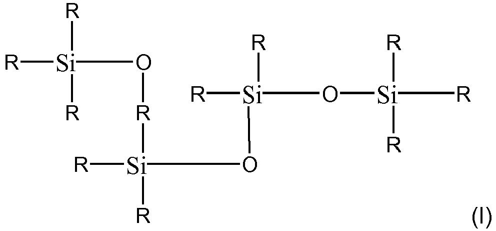 in which the groups R, which are monovalent or divalent, represent, independently of each other, a methyl, methylene, phenyl or phenylene, with the proviso that at least one group R represents a phenyl.

Preferably, in this formula, the phenyl silicone oil comprises at least three phenyl groups, for example at least four, at least five or at least six.
- phenyl silicone oils optionally having a dimethicone fragment corresponding to formula (II) below: 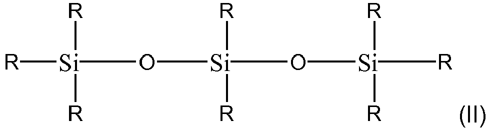 in which the groups R represent, independently of each other, a methyl or a phenyl, with the proviso that at least one group R represents a phenyl.

Preferably, in this formula, the compound of formula (II) comprises at least three phenyl groups, for example at least four or at least five.

Mixtures of different phenylorganopolysiloxane compounds described above can be used.

Examples which may be mentioned comprise mixtures of triphenyl-, tetraphenyl- or pentaphenylorganopolysiloxanes.

Mention may more particularly be made, among the compounds of formula (II), of phenyl silicone oils not having a dimethicone fragment, corresponding to the formula (II) in which at least 4 or at least 5 R radicals represent a phenyl radical, the remaining radicals representing methyls.

Such non-volatile phenyl silicone oils are preferably trimethylpentaphenyltrisiloxane or tetramethyltetraphenyltrisiloxane. They are in particular sold by Dow Corning under the reference PH-1555 HRI or Dow Corning 555 Cosmetic Fluid (chemical name: 1,3,5-trimethyl-1,1,3,5,5-pentaphenyltrisiloxane; INCI name: trimethylpentaphenyltrisiloxane), or the tetramethyltetraphenyltrisiloxane sold under the reference Dow Corning 554 Cosmetic Fluid by Dow Corning can also be used.

They correspond in particular to formulae (III) and (III') below: 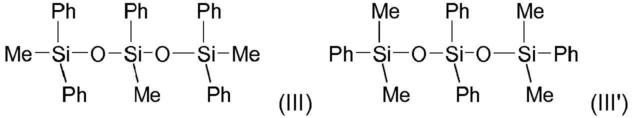 in which Me represents methyl, and Ph represents phenyl.
- phenyl silicone oils having at least one dimethicone fragment corresponding to formula (IV) below: 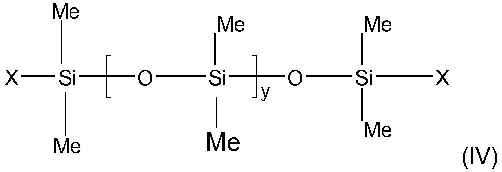 in which Me represents methyl, y is between 1 and 1000 and X represents -CH₂-CH(CH₃)(Ph).
- phenyl silicone oils corresponding to formula (V) below, and mixtures thereof: 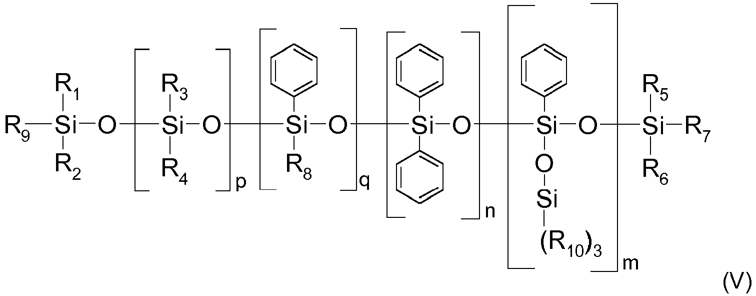 in which:
   - R₁ to R₁₀, independently of one another, are saturated or unsaturated and linear, cyclic or branched C₁-C₃₀ hydrocarbon-based radicals,
   - m, n, p and q are, independently of each other, integers between 0 and 900, with the proviso that the sum m+n+q is other than 0.

Preferably, the sum m+n+q is between 1 and 100. Advantageously, the sum m+n+p+q is between 1 and 900 and preferably between 1 and 800.
Preferably, q is equal to 0.

More particularly, R₁ to R₁₀ represent, independently of one another, a saturated or unsaturated, preferably saturated, and linear or branched C₁-C₃₀ hydrocarbon-based radical, and in particular a preferably saturated C₁-C₂₀, in particular C₁-C₁₈, hydrocarbon-based radical, or a monocyclic or polycyclic C₆-C₁₄ and in particular C₁₀-C₁₃ aryl radical, or an aralkyl radical, the alkyl part of which is preferably a C₁-C₃ alkyl part.

Preferably, R₁ to R₁₀ can each represent a methyl, ethyl, propyl, butyl, isopropyl, decyl, dodecyl or octadecyl radical, or in an alternative form a phenyl, tolyl, benzyl or phenethyl radical. R₁ to R₁₀ can in particular be identical, and in addition can be a methyl radical.

As particular embodiments of formula (V), mention may be made of:
∘ phenyl silicone oils optionally having at least one dimethicone fragment corresponding to formula (VI) below, and mixtures thereof: 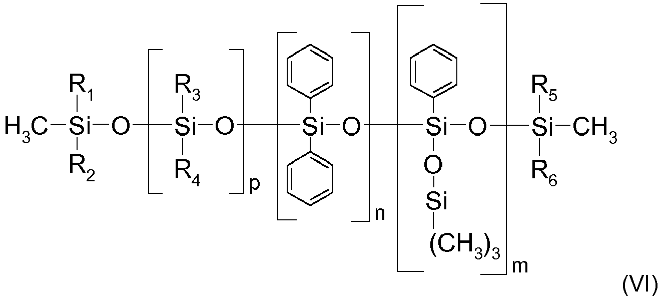 in which:
   ▪ R₁ to R₆, independently of each other, are saturated or unsaturated, linear, cyclic or branched C₁-C₃₀ hydrocarbon-based radicals, a preferably C₆-C₁₄ aryl radical or an aralkyl radical, the alkyl part of which is C₁-C₃ alkyl,
   ▪ m, n and p are, independently of each other, integers between 0 and 100, with the proviso that the sum n+m is between 1 and 100.

Preferably, R₁ to R₆ represent, independently of one another, a C₁-C₂₀, in particular C₁-C₁₈, hydrocarbon-based, preferably alkyl, radical, or a C₆-C₁₄ aryl radical which is monocyclic (preferably a C₆ aryl radical) or polycyclic and in particular a C₁₀-C₁₃ aryl radical, or an aralkyl radical (preferably the aryl part is a C₆ aryl part; the alkyl part is a C₁-C₃ alkyl part).

Preferably, R₁ to R₆ can each represent a methyl, ethyl, propyl, butyl, isopropyl, decyl, dodecyl or octadecyl radical, or in an alternative form a phenyl, tolyl, benzyl or phenethyl radical.

R₁ to R₆ can in particular be identical, and in addition can be a methyl radical. Preferably, m = 1 or 2 or 3, and/or n = 0 and/or p = 0 or 1 may be applied, in formula (VI).

According to a particular embodiment, the non-volatile phenyl silicone oil is chosen from phenyl silicone oils bearing at least one dimethicone fragment.

Preferably, such oils correspond to compounds of formula (VI) in which:
▪ m=0 and n and p are, independently of each other, integers between 1 and 100.

Preferably, R₁ to R₆ are methyl radicals.

According to this embodiment, the silicone oil is preferably chosen from a diphenyl dimethicone, such as KF-54 from Shin-Etsu (400 cSt), KF54HV from Shin-Etsu (5000 cSt), KF-50-300CS from Shin-Etsu (300 cSt), KF-53 from Shin-Etsu (175 cSt) or KF-50-100CS from Shin-Etsu (100 cSt).
▪ p is between 1 and 100, the sum n+m is between 1 and 100, and n=0.

These phenyl silicone oils optionally have at least one dimethicone fragment corresponding more particularly to formula (VII) below: 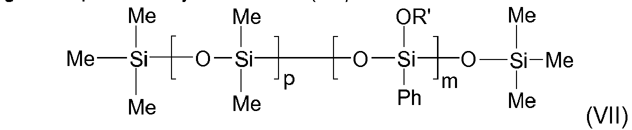 in which Me is methyl and Ph is phenyl, OR' represents an -OSiMe₃ group and p is 0 or is between 1 and 1000, and m is between 1 and 1000. In particular, m and p are such that compound (VII) is a non-volatile oil.
According to a first embodiment of non-volatile phenyl silicone having at least one dimethicone fragment, p is between 1 and 1000 and m is more particularly such that the compound (VII) is a non-volatile oil. Use may be made, for example, of trimethylsiloxyphenyl dimethicone, sold in particular under the reference Belsil PDM 1000 by Wacker.

According to a second embodiment of non-volatile phenyl silicone not having a dimethicone fragment, p is equal to 0 and m is between 1 and 1000, and in particular is such that the compound (VII) is a non-volatile oil.

Phenyltrimethylsiloxytrisiloxane, sold in particular under the reference Dow Corning 556 Cosmetic Grade Fluid (DC556), can be used, for example.
∘ non-volatile phenyl silicone oils not having a dimethicone fragment corresponding to formula (VIII) below, and mixtures thereof: 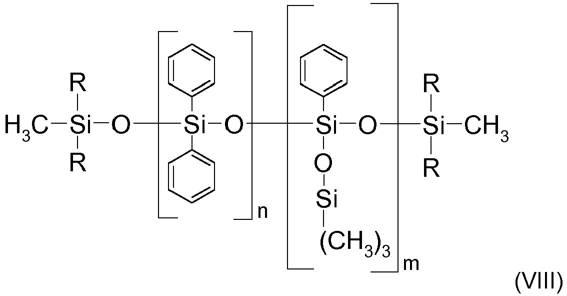 in which:
   - R, independently of each other, are saturated or unsaturated, linear, cyclic or branched C₁-C₃₀ hydrocarbon-based radicals, preferably R is a C₁-C₃₀ alkyl radical, a preferably C₆-C₁₄ aryl radical, or an aralkyl radical, the alkyl part of which is C₁-C₃ alkyl,
   - m and n are, independently of each other, integers between 0 and 100, with the proviso that the sum n+m is between 1 and 100.

Preferably, R, independently of each other, represent a saturated or unsaturated, preferably saturated, linear or branched C₁-C₃₀ hydrocarbon-based radical, and in particular a preferably saturated, C₁-C₂₀, in particular C₁-C₁₈ and more particularly C₄-C₁₀, hydrocarbon-based radical, a monocyclic or polycyclic C₆-C₁₄, and in particular C₁₀-C₁₃, aryl radical, or an aralkyl radical of which preferably the aryl part is C₆ aryl and the alkyl part is C₁-C₃ alkyl.

Preferably, the R groups can each represent a methyl, ethyl, propyl, butyl, isopropyl, decyl, dodecyl or octadecyl radical, or in an alternative form a phenyl, tolyl, benzyl or phenethyl radical.

The R groups can in particular be identical, and in addition can be a methyl radical.

Preferably, m = 1 or 2 or 3, and/or n = 0 and/or p = 0 or 1 may be applied, in formula (VIII).

According to one preferred embodiment, n is an integer between 0 and 100 and m is an integer between 1 and 100, with the proviso that the sum n+m is between 1 and 100, in formula (VIII). Preferably, R is a methyl radical.

According to one embodiment, a phenyl silicone oil of formula (VIII) with a viscosity at 25°C of between 5 and 1500 mm²/s (i.e. 5 to 1500 cSt), and preferably with a viscosity of between 5 and 1000 mm²/s (i.e. 5 to 1000 cSt), may be used.

According to this embodiment, the non-volatile phenyl silicone oil is preferably chosen from phenyl trimethicones (when n=0) such as DC556 from Dow Corning (22.5 cSt), or else from diphenylsiloxyphenyl trimethicone oil (when m and n are between 1 and 100) such as KF56 A from Shin-Etsu, or the Silbione 70663V30 oil from Rhône-Poulenc (28 cSt). The values in brackets represent the viscosities at 25°C.
- phenyl silicone oils optionally having at least one dimethicone fragment corresponding to the following formula, and mixtures thereof: 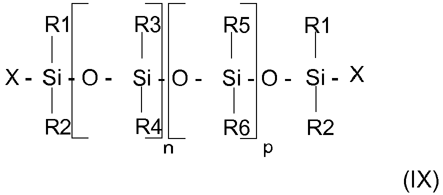 in which:
   R₁, R₂, R₅ and R₆, which are identical or different, are an alkyl radical containing from 1 to 6 carbon atoms,
   R₃ and R₄, which are identical or different, are an alkyl radical containing from 1 to 6 carbon atoms or an aryl radical (preferably a C₆-C₁₄ aryl radical), with the proviso that at least one of R₃ and R₄ is a phenyl radical,
   X is an alkyl radical containing from 1 to 6 carbon atoms, a hydroxyl radical or a vinyl radical, n and p being an integer greater than or equal to 1, chosen so as to give the oil a weight-average molecular weight preferably less than 150 000 g/mol and more preferably less than 100 000 g/mol.
- and a mixture thereof.

Preferably, the composition used in the context of the present invention comprises non-volatile phenyl silicone oil(s) not having a dimethicone fragment.

More particularly, the non-volatile phenyl silicone oils not having a dimethicone fragment are chosen from (I), with radicals R such that the silicone has no dimethicone fragment; (II) with radicals R such that the silicone has no dimethicone fragment, in particular formulae (III) and (III'); (V) with p = 0; (VI) with p=0; (VII) with p=0; (VIII); (IX) with radicals R such that the silicone has no dimethicone fragment; or mixtures thereof.

Furthermore, preferably, the non-volatile phenyl silicone oils are chosen from those of formula (II), more particularly non-volatile phenyl silicone oils of formula (III) or (III').

In accordance with a particular embodiment of the invention, the content of non-volatile phenyl silicone oil(s) preferably not having a dimethicone fragment ranges from 1% to 8% by weight, preferably from 1.5% to 5% by weight, relative to the weight of the composition.

### ADDITIONAL NON-VOLATILE OILS

### Non-volatile non-phenyl silicone oils

The composition may optionally comprise at least one additional non-volatile oil, chosen from non-volatile non-phenyl silicone oils.

The expression "non-phenyl silicone oil" denotes a silicone oil not comprising phenyl substituents.

Representative examples of these non-volatile non-phenyl silicone oils which can be mentioned comprise polydimethylsiloxanes; alkyl dimethicones; vinyl methyl methicones; and also silicones modified with aliphatic groups and/or with functional groups, such as hydroxyl, thiol and/or amine groups, preferably hydroxyl groups.

It should be noted that "dimethicone" (INCI name) corresponds to a polydimethylsiloxane (chemical name).

In particular, these oils can be chosen from the following non-volatile oils:
- polydimethylsiloxanes (PDMSs),
- alkyl dimethicones comprising aliphatic groups, in particular alkyl or alkoxy groups, which are pendent and/or at the end of the silicone chain, these groups each comprising from 2 to 24 carbon atoms. Mention may be made, by way of example, of cetyl dimethicone, sold under the commercial reference Abil Wax 9801 from Evonik Goldschmidt,
- PDMSs comprising functional groups, such as hydroxyl, thiol and/or amine groups, preferably hydroxyl groups,
- polydimethylsiloxanes substituted with aliphatic groups, in particular C₂-C₂₄ groups, which are pendent and/or at the end of the silicone chain, and functional groups such as hydroxyl, thiol and/or amine groups, preferably hydroxyl groups,
- polysiloxanes modified with fatty acids or fatty alcohols, and
- mixtures thereof.

Preferably, these non-volatile non-phenyl silicone oils are chosen from polydimethylsiloxanes; alkyl dimethicones and also polydimethylsiloxanes substituted with aliphatic groups, in particular C₂-C₂₄ alkyl groups, and functional groups such as hydroxyl groups.

The non-volatile non-phenyl silicone oil can be chosen in particular from silicones of formula (I): 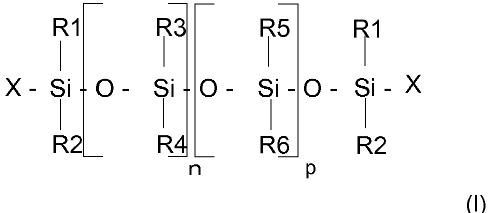 in which:
- R₁, R₂, R₅ and R₆ are, together or separately, an alkyl radical containing 1 to 6 carbon atoms,
- R₃ and R₄ are, together or separately, an alkyl radical containing 1 to 6 carbon atoms, or a hydroxyl radical,
- X is an alkyl radical containing from 1 to 6 carbon atoms, a hydroxyl radical,
- n and p are integers chosen so as to have a fluid compound, the viscosity of which at 25°C is in particular between 8 centistokes (cSt) (8 × 10⁻⁶ m²/s) and 100 000 cSt, and advantageously a weight-average molecular weight of less than or equal to 150 000 g/mol, preferably of less than or equal to 100 000 g/mol and better still of less than or equal to 10 000 g/mol.

There may be mentioned, as non-volatile non-phenyl silicone oils suitable for the implementation of the invention, those for which:
- the R₁ to R₆ and X substituents represent a methyl group, and p and n are such that the viscosity is 60 000 cSt, for example the product sold under the name Dow Corning 200 Fluid 60 000 CS by Dow Corning and the product sold under the name Wacker Belsil DM 60 000 by Wacker,
- the R₁ to R₆ and X substituents represent a methyl group, and p and n are such that the viscosity is 100 cSt or 350 cSt, for example the products sold respectively under the names Belsil DM100 and Dow Corning 200 Fluid 350 CS by Dow Corning, and
- the R₁ to R₆ substituents represent a methyl group, the X group represents a hydroxyl group, and n and p are such that the viscosity is 700 cSt, for example the product sold under the name Baysilone Fluid T0.7 by Momentive.

### Non-polar non-volatile hydrocarbon-based oils

The composition according to the invention may also comprise at least one additional non-polar non-volatile hydrocarbon-based oil.

These oils can be of plant, mineral or synthetic origin.

The term "non-polar hydrocarbon-based oil" is intended to mean, within the meaning of the present invention, an oil comprising only carbon and hydrogen atoms in its structure.

More particularly, the non-polar non-volatile hydrocarbon-based oils are chosen from linear or branched hydrocarbons of mineral or synthetic origin, such as:
- liquid paraffin or derivatives thereof (mineral oil),
- squalane,
- isoeicosane,
- naphthalene oil,
- polybutenes, such as, for example, Indopol H-100, Indopol H-300 or Indopol H-1500 sold by the company Amoco,
- polyisobutenes and hydrogenated polyisobutenes, such as, in particular, Parleam^{®} products sold by the company Nippon Oil Fats, Panalane H-300 E sold by the company Amoco, Viseal 20000 sold by the company Synteal, Rewopal P!B 1000 sold by the company Witco or alternatively Parleam Lite sold by NOF Corporation,
- decene/butene copolymers, polybutene/polyisobutene copolymers, especially Indopol L-14,
- polydecenes and hydrogenated polydecenes, such as, in particular: Puresyn 10, Puresyn 150 or alternatively Puresyn 6 sold by the company ExxonMobil Chemicals,
- and mixtures thereof.

If they are present in the composition used, then their content is such that the total content of non-volatile oils (in other words polar or non-polar, or phenyl or non-phenyl silicone hydrocarbon-based oils, having or not having at least one dimethicone fragment) varies between 6% and 20% by weight, preferably between 6% and 15% by weight, relative to the weight of the composition.

### Non-volatile fluorinated oils

The term *"fluorinated oil"* is intended to mean an oil containing at least one fluorine atom.

As examples of fluorinated oils, mention may be made of fluorosilicone oils, fluorinated polyethers, fluorosilicones in particular as described in document EP-A-847 752 and perfluorinated compounds.

Perfluorinated compounds is intended to mean, according to the invention, compounds in which all the hydrogen atoms have been replaced by fluorine atoms.

According to a preferred embodiment, the fluorinated oil is chosen from perfluorinated oils.

As examples of perfluorinated oils, mention may be made of perfluorodecalins and perfluoroperhydrophenanthrenes.

According to a preferred embodiment, the fluorinated oil is chosen from perfluoroperhydrophenanthrenes and in particular the Fiflow^{®} products sold by Créations Couleurs. In particular, use may be made of the fluorinated oil for which the INCI name is Perfluoroperhydrophenanthrene, sold under the reference Fiflow 220 by the company F2 Chemicals.

### FILM-FORMING AGENT COMPRISING CARBOSILOXANE DENDRIMER UNIT(S)

Moreover, the composition according to the invention comprises at least one film-forming agent chosen from vinyl polymers comprising at least one carbosiloxane dendrimer-based unit.

More particularly, the content of film-forming agent(s) represents from 0.5% to 30% by weight of active material and preferably from 1% to 20% by weight, relative to the weight of the composition.

The term "film-forming" polymer is intended to mean a polymer that is capable of forming, by itself or in the presence of an auxiliary film-forming agent, a continuous deposit on a support, especially on keratin materials.

The vinyl polymer(s) have a backbone and at least one side chain, which comprises a carbosiloxane dendrimer-based unit having a carbosiloxane dendrimer structure.

In the context of the present invention, the term "carbosiloxane dendrimer structure" represents a molecular structure possessing branched groups having high molecular weights, said structure having high regularity in the radial direction starting from the bond to the backbone. Such carbosiloxane dendrimer structures are described in the form of a highly branched siloxane-silylalkylene copolymer in Japanese patent application JP 9-171 154.

A vinyl polymer according to the invention may contain units derived from carbosiloxane dendrimers that may be represented by the following general formula (I): 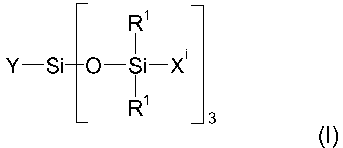 in which:
- R¹ represents an aryl group having from 5 to 10 carbon atoms or an alkyl group having from 1 to 10 carbon atoms;
- Xⁱ represents a silylalkyl group which, when i = 1, is represented by formula (II): 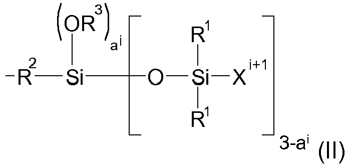 in which:
   . R¹ is as defined above in the formula (I),
   . R² represents an alkylene radical having from 2 to 10 carbon atoms,
   . R³ represents an alkyl group having from 1 to 10 carbon atoms,
   . Xⁱ⁺ⁱ is chosen from: a hydrogen atom, an alkyl group containing from 1 to 10 carbon atoms, an aryl group containing from 5 to 10 carbon atoms and a silylalkyl group defined above of formula (II) with i = i + 1,
   . i is an integer from 1 to 10 which represents the generation of said silylalkyl group, and
   . aⁱ is an integer from 0 to 3;
   - Y represents a radically polymerizable organic group chosen from:
      . organic groups comprising a methacrylic group or an acrylic group, said organic groups being represented by the formulae: 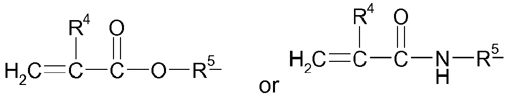 in which:
      * R⁴ represents a hydrogen atom or an alkyl group having from 1 to 10 carbon atoms; and
      * R⁵ represents an alkylene group having from 1 to 10 carbon atoms, such as a methylene group, an ethylene group, a propylene group or a butylene group, methylene and propylene groups being preferred; and
      . organic groups comprising a styryl group of formula: 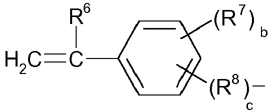 in which:
         _{*} R⁶ represents a hydrogen atom or an alkyl group having from 1 to 10 carbon atoms, such as a methyl group, an ethyl group, a propyl group or a butyl group, the methyl group being preferred;
         * R⁷ represents an alkyl group having from 1 to 10 carbon atoms;
         * R⁸ represents an alkylene group having from 1 to 10 carbon atoms, such as a methylene group, an ethylene group, a propylene group or a butylene group, the ethylene group being preferred;
         * b is an integer from 0 to 4; and
         * c is 0 or 1, such that, if c is 0, -(R⁸)_{c}- represents a bond.

According to one embodiment, R¹ can represent an aryl group possessing from 5 to 10 carbon atoms or an alkyl group possessing from 1 to 10 carbon atoms. The alkyl group can preferably be represented by a methyl group, an ethyl group, a propyl group, a butyl group, a pentyl group, an isopropyl group, an isobutyl group, a cyclopentyl group or a cyclohexyl group. The aryl group can preferably be represented by a phenyl group and a naphthyl group. The methyl and phenyl groups are more particularly preferred, and the methyl group is most preferred.

According to one embodiment, R² represents an alkylene group possessing from 2 to 10 carbon atoms, in particular a linear alkylene group, such as an ethylene, propylene, butylene or hexylene group; or a branched alkylene group, such as a methylmethylene, methylethylene, 1-methylpentylene or 1,4-dimethylbutylene group.

The ethylene, methylethylene, hexylene, 1-methylpentylene and 1,4-dimethylbutylene groups are most preferred.

According to one embodiment, R³ is chosen from methyl, ethyl, propyl, butyl and isopropyl groups.

In the formula (II), i indicates the number of generations and thus corresponds to the number of repetitions of the silylalkyl group.

For example, when the number of generations is equal to 1, the carbosiloxane dendrimer can be represented by the general formula shown below, in which Y, R¹, R² and R³ are as defined above, R¹² represents a hydrogen atom or is identical to R¹ and a¹ is identical to aⁱ. Preferably, the total average number of OR³ groups in a molecule is within the range from 0 to 7.

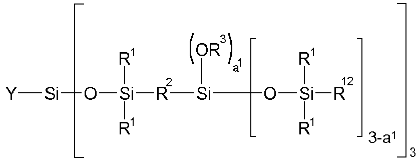

When the generation number is equal to 2, the carbosiloxane dendrimer may be represented by the general formula below, in which Y, R¹, R², R³ and R¹² are the same as defined above; a¹ and a² represent the aⁱ of the indicated generation. Preferably, the total average number of OR³ groups in a molecule is within the range from 0 to 25.

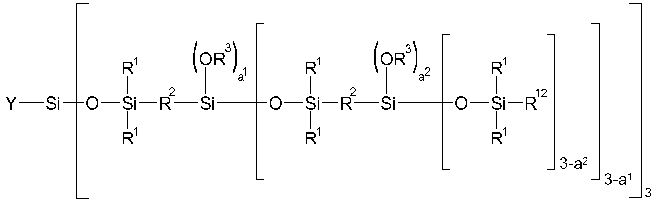

When the generation number is equal to 3, the carbosiloxane dendrimer is represented by the general formula below, in which Y, R¹, R², R³ and R¹² are the same as defined above; a¹, a² and a³ represent the aⁱ of the indicated generation. Preferably, the total average number of OR³ groups in a molecule is within the range from 0 to 79.

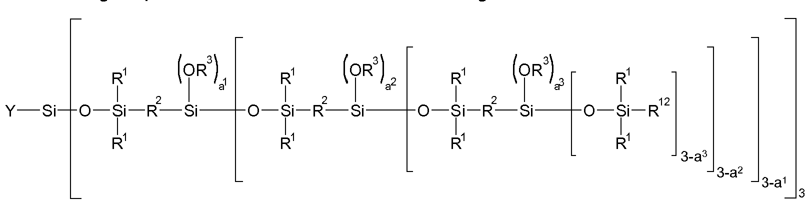

A vinyl polymer having at least one unit derived from carbosiloxane dendrimer has a molecular side chain containing a carbosiloxane dendrimer structure and can result from the polymerization of:
(A) from 0 to 99.9 parts by weight of a vinyl monomer, and
(B) from 100 to 0.1 parts by weight of a carbosiloxane dendrimer comprising a radically polymerizable organic group, represented by the general formula (I) as defined above.

The monomer of vinyl type which is the component (A) in the vinyl polymer having at least one unit derived from carbosiloxane dendrimer is a monomer of vinyl type which comprises a radically polymerizable vinyl group.

There is no particular limitation as regards such a monomer.

The following are examples of this monomer of vinyl type: methyl methacrylate, ethyl methacrylate, n-propyl methacrylate, isopropyl methacrylate or a methacrylate of lower alkyl analogue; glycidyl methacrylate; butyl methacrylate, butyl acrylate, n-butyl methacrylate, isobutyl methacrylate, tert-butyl acrylate, tert-butyl methacrylate, n-hexyl methacrylate, cyclohexyl methacrylate, 2-ethylhexyl acrylate, 2-ethylhexyl methacrylate, octyl methacrylate, lauryl methacrylate, stearyl acrylate, stearyl methacrylate or a higher methacrylate analogue; vinyl acetate, vinyl propionate or a vinyl ester of a lower fatty acid analogue; vinyl caproate, vinyl 2-ethylhexoate, vinyl laurate, vinyl stearate or a higher fatty acid ester analogue; styrene, vinyltoluene, benzyl methacrylate, phenoxyethyl methacrylate, vinylpyrrolidone or similar vinylaromatic monomers; methacrylamide, N-methylolmethacrylamide, N-methoxymethyl methacrylamide, isobutoxymethoxymethacrylamide, N,N-dimethylmethacrylamide or similar monomers of vinyl type containing amide groups; hydroxyethyl methacrylate, hydroxypropyl alcohol methacrylate or similar monomers of vinyl type containing hydroxyl groups; acrylic acid, methacrylic acid, itaconic acid, crotonic acid, fumaric acid, maleic acid or similar monomers of vinyl type containing a carboxylic acid group; tetrahydrofurfuryl methacrylate, butoxyethyl methacrylate, ethoxydiethylene glycol methacrylate, polyethylene glycol methacrylate, polypropylene glycol monomethacrylate, hydroxybutyl vinyl ether, cetyl vinyl ether, 2-ethylhexyl vinyl ether or a similar monomer of vinyl type with ether bonds; methacryloxypropyltrimethoxysilane, polydimethylsiloxane containing a methacrylic group on one of its molecular ends, polydimethylsiloxane containing a styryl group on one of its molecular ends, or a similar silicone compound containing unsaturated groups; butadiene; vinyl chloride; vinylidene chloride; methacrylonitrile; dibutyl fumarate; anhydrous maleic acid; anhydrous succinic acid; methacryl glycidyl ether; an organic salt of an amine, an ammonium salt, and an alkali metal salt of methacrylic acid, of itaconic acid, of crotonic acid, of maleic acid or of fumaric acid; a radically polymerizable unsaturated monomer containing a sulfonic acid group such as a styrenesulfonic acid group; a quaternary ammonium salt derived from methacrylic acid, such as 2-hydroxy-3-methacryloxypropyltrimethylammonium chloride; and a methacrylic acid ester of an alcohol containing a tertiary amine group, such as a methacrylic acid ester of diethylamine.

Multifunctional monomers of vinyl type can also be used.

The following represent examples of such compounds: trimethylolpropane trimethacrylate, pentaerythrityl trimethacrylate, ethylene glycol dimethacrylate, tetraethylene glycol dimethacrylate, polyethylene glycol dimethacrylate, 1,4-butanediol dimethacrylate, 1,6-hexanediol dimethacrylate, neopentyl glycol dimethacrylate, ethoxylated trimethylolpropane trimethacrylate, tris(2-hydroxyethyl)isocyanurate dimethacrylate, tris(2-hydroxyethyl)isocyanurate trimethacrylate, polydimethylsiloxane capped with styryl groups possessing divinylbenzene groups on both ends, or analogous silicone compounds containing unsaturated groups.

A carbosiloxane dendrimer, which is the component (B), can be represented by the formula (I) as defined above.

The following represent the preferred examples of Y group of the formula (I): an acryloyloxymethyl group, a 3-acryloyloxypropyl group, a methacryloyloxymethyl group, a 3-methacryloyloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, a vinyl group, an allyl group, a methallyl group and a 5-hexenyl group.

A carbosiloxane dendrimer according to the present invention may be represented by the formulae having the average structures below:

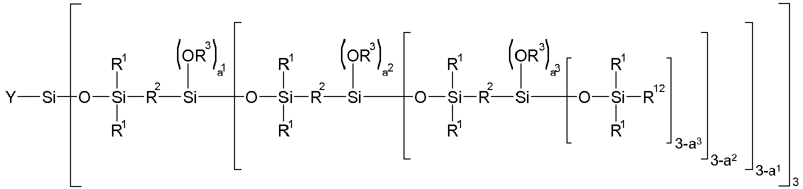

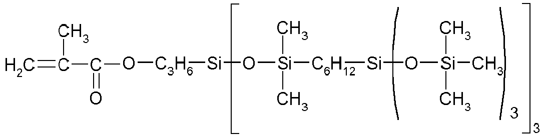

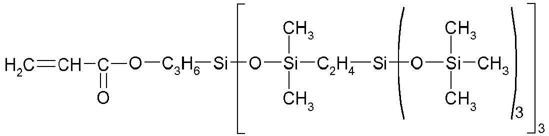

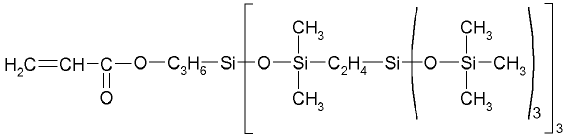

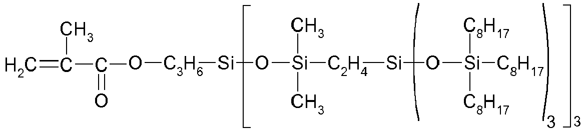

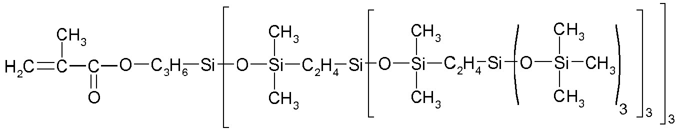

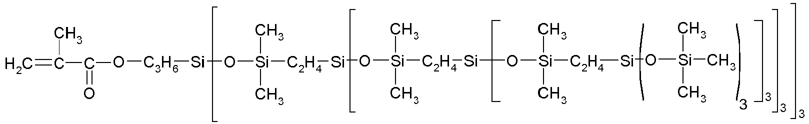

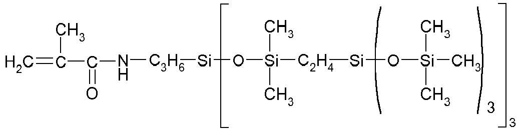

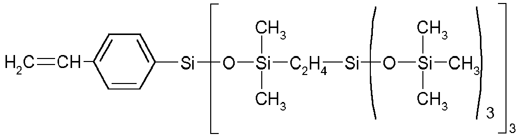

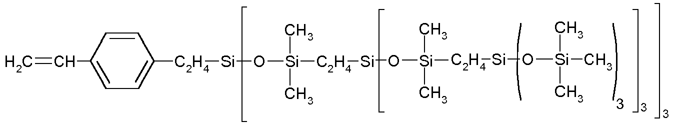

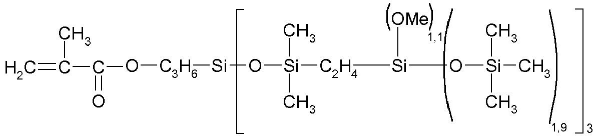

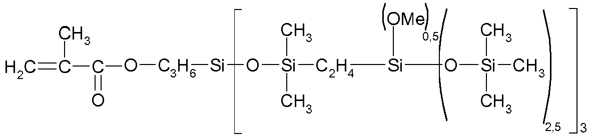

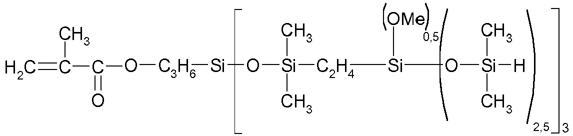

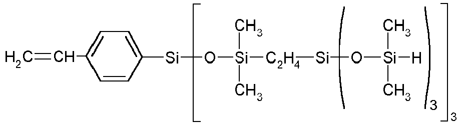

Thus, according to one embodiment, the carbosiloxane dendrimer of the composition according to the present invention is represented by the following formula: 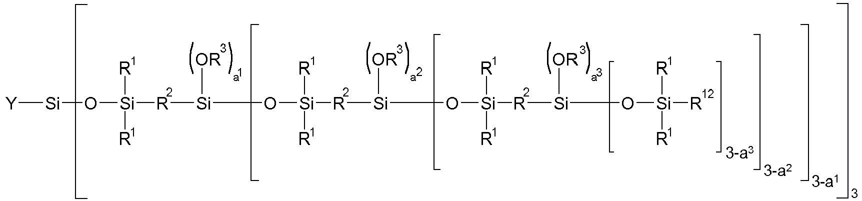 in which:
. Y, R¹, R² and R³ are as defined in the formulae (I) and (II) above;
. a¹, a² and a³ correspond to the definition of aⁱ according to formula (II); and
. R¹² is H, an aryl group having from 5 to 10 carbon atoms or an alkyl group having from 1 to 10 carbon atoms.

According to one embodiment, the carbosiloxane dendrimer of the composition according to the present invention is represented by one of the following formulae:

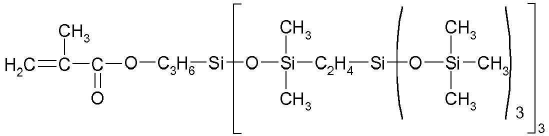

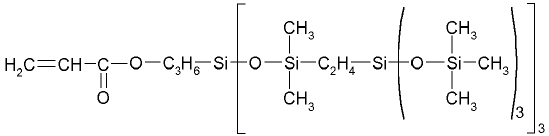

The vinyl polymer comprising the carbosiloxane dendrimer according to the invention can be manufactured according to the process for manufacturing a branched silalkylene siloxane described in Japanese Patent Application Hei 9-171 154.

To facilitate the preparation of starting material for cosmetic products, the number-average molecular weight of the vinyl polymer which comprises a carbosiloxane dendrimer can be chosen within the range between 3000 and 2 000 000 g/mol and preferably between 5000 and 800 000 g/mol. It can be a liquid, a gum, a paste, a solid, a powder or any other form. The preferred forms are solutions formed by the dilution of a dispersion or of a powder in solvents.

The vinyl polymer can be a dispersion of a polymer of vinyl type having a carbosiloxane dendrimer structure in its molecular side chain, in a liquid such as a silicone oil, an organic oil, an alcohol or water.

The silicone oil can be a dimethylpolysiloxane having the two molecular ends capped with trimethylsiloxy groups, a copolymer of methylphenylsiloxane and of dimethylsiloxane having the two molecular ends capped with trimethylsiloxy groups, a copolymer of methyl(3,3,3-trifluoropropyl)siloxane and of dimethylsiloxane having the two molecular ends capped with trimethylsiloxy groups, or analogous unreactive linear silicone oils, and also hexamethylcyclotrisiloxane, octamethylcyclotetrasiloxane, decamethylcyclopentasiloxane, dodecamethylcyclohexasiloxane or an analogous cyclic compound. In addition to the unreactive silicone oils, modified polysiloxanes containing functional groups such as silanol groups, amino groups and polyether groups on the ends or within the molecular side chains may be used.

The organic oils can be isododecane, liquid paraffin, isoparaffin, hexyl laurate, isopropyl myristate, myristyl myristate, cetyl myristate, 2-octyldodecyl myristate; isopropyl palmitate, 2-ethylhexyl palmitate, butyl stearate, decyl oleate, 2-octyldodecyl oleate, myristyl lactate, cetyl lactate, lanolin acetate, stearyl alcohol, cetostearyl alcohol, oleyl alcohol, avocado oil, almond oil, olive oil, cocoa oil, jojoba oil, gum oil, sunflower oil, soybean oil, camelia oil, squalane, castor oil, cottonseed oil, coconut oil, egg yolk oil, polypropylene glycol monooleate, neopentyl glycol 2-ethylhexanoate or an analogous glycol ester oil; triglyceryl isostearate, the triglyceride of a fatty acid of coconut oil, or an analogous oil of a polyhydric alcohol ester; polyoxyethylene lauryl ether, polyoxypropylene cetyl ether or an analogous polyoxyalkylene ether.

The alcohol may be any type that is suitable for use in combination with a cosmetic product starting material. For example, it can be methanol, ethanol, butanol, isopropanol or lower alcohol analogues.

A solution or a dispersion of the alcohol should have a viscosity within the range from 10 to 10⁹ mPa at 25°C. To improve the sensory use properties in a cosmetic product, the viscosity should be within the range from 100 to 5 × 10⁸ mPa.s.

The solutions and dispersions can be easily prepared by mixing a vinyl polymer having at least one unit derived from carbosiloxane dendrimer with a silicone oil, an organic oil, an alcohol or water. The liquids can be present in the polymerization stage. In this case, the unreacted residual vinyl monomer should be completely removed by heat treatment of the solution or dispersion under atmospheric pressure or reduced pressure.

In the case of a dispersion, the dispersity of the polymer of vinyl type can be improved by adding a surfactant.

Such a surfactant can be hexylbenzenesulfonic acid, octylbenzenesulfonic acid, decylbenzenesulfonic acid, dodecylbenzenesulfonic acid, cetylbenzenesulfonic acid, myristylbenzenesulfonic acid or anionic surfactants of the sodium salts of these acids; octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, (beef tallow)trimethylammonium hydroxide, (coconut oil)trimethylammonium hydroxide, or an analogous cationic surfactant; a polyoxyalkylene alkyl ether, a polyoxyalkylene alkylphenol, a polyoxyalkylene alkyl ester, the sorbitol ester of polyoxyalkylene, polyethylene glycol, polypropylene glycol, an ethylene oxide additive of diethylene glycol trimethylnonanol, and non-ionic surfactants of polyester type, and also mixtures.

In the dispersion, a mean particle diameter of the polymer of vinyl type can be within a range of between 0.001 and 100 microns and preferably between 0.01 and 50 microns. The reason for this is that, outside the recommended range, a cosmetic product mixed with the emulsion will not have a nice enough feel on the lips or to the touch, nor sufficient spreading properties nor a pleasant feel.

A vinyl polymer contained in the dispersion or the solution may have a concentration in the range between 0.1% and 95% by weight and preferably between 5% and 85% by weight. However, to facilitate the handling and the preparation of the mixture, the range should preferably be between 10% and 75% by weight.

A vinyl polymer suitable for the invention can also be one of the polymers described in the examples of patent application EP 0 963 751.

According to a preferred embodiment, a vinyl polymer grafted with a carbosiloxane dendrimer may be the product of polymerization of:
(A1) from 0 to 99.9 parts by weight of one or more acrylate or methacrylate monomer(s); and
(B1) from 100 to 0.1 parts by weight of an acrylate or methacrylate monomer of a tris[tri(trimethylsiloxy)silylethyldimethylsiloxy]silylpropyl carbosiloxane dendrimer.

The monomers (A1) and (B1) correspond respectively to specific monomers (A) and (B).

According to one embodiment, a vinyl polymer bearing at least one carbosiloxane dendrimer-based unit may comprise a tris[tri(trimethylsiloxy)silylethyldimethylsiloxy]silylpropyl carbosiloxane dendrimer-based unit corresponding to one of the formulae: 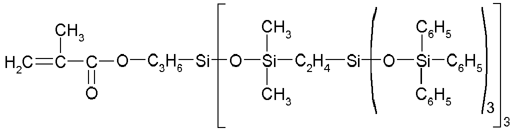 or

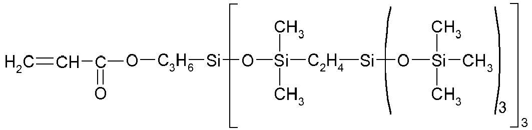

According to a preferred mode, a vinyl polymer bearing at least one carbosiloxane dendrimer-based unit used in the invention comprises at least one butyl acrylate monomer.

According to one embodiment, a vinyl polymer may also comprise at least one fluorinated organic group.

Structures in which the polymerized vinyl units constitute the backbone and carbosiloxane dendritic structures and also fluorinated organic groups are attached to side chains are particularly preferred.

The fluorinated organic groups can be obtained by replacing with fluorine atoms all or some of the hydrogen atoms of methyl, ethyl, propyl, isopropyl, butyl, isobutyl, tert-butyl, pentyl, neopentyl, hexyl, cyclohexyl, heptyl, octyl, nonyl, decyl, undecyl, dodecyl, tridecyl, tetradecyl, hexadecyl and octadecyl groups and of other alkyl groups of 1 to 20 carbon atoms, and also alkyloxyalkylene groups of 6 to 22 carbon atoms.

The groups represented by the formula -(CH₂)ₓ-(CF₂)_{y}-R¹³ are suggested as examples of fluoroalkyl groups obtained by substituting fluorine atoms for hydrogen atoms of alkyl groups. In the formula, the subscript "x" is 0, 1, 2 or 3, and "y" is an integer from 1 to 20. R¹³ is an atom or a group chosen from a hydrogen atom, a fluorine atom, -CH(CF₃)₂- or CF(CF₃)₂. Such fluorine-substituted alkyl groups are exemplified by linear or branched polyfluoroalkyl or perfluoroalkyl groups represented by the formulae presented below: -CF₃, -C₂F₅, -nC₃F₇, -CF(CF₃)₂, -nC₄F₉, CF₂CF(CF₃)₂, -nC₅F₁₁, -nC₆F₁₃, -nC₈F₁₇, CH₂CF₃, -(CH(CF₃)₂, CH₂CH(CF₃)₂-CH₂(CF₂)₂F, -CH₂(CF₂)₃F, -CH₂(CF₂)₄F,CH₂(CF₂)₆F, CH₂(CF₂)₈F, -CH₂CH₂CF₃, -CH₂CH₂(CF₂)₂F, -CH₂CH₂(CF₂)₃F, -CH₂CH₂(CF₂)₄F, -CH₂CH₂(CF₂ )₆F, -CH₂CH₂(CF₂)₈F, -CH₂CH₂(CF₂)₁₀F, -CH₂CH₂(CF₂)₁₂F, CH₂CH₂(CF₂)₁₄F,-CH₂CH₂(CF₂)₁₆F, -CH₂CH₂CH₂CF₃, -CH₂CH₂CH₂(CF₂)₂F, -CH₂CH₂CH₂(CF₂ )₂H, -CH₂(CF₂)₄H and -CH₂CH₂(CF₂)₃H.

The groups represented by -CH₂CH₂-(CF₂)ₘ-CFR¹⁴-[OCF₂CF(CF₃)]ₙ-OC₃F₇ are suggested as fluoroalkyloxyfluoroalkylene groups obtained by substituting fluorine atoms for hydrogen atoms of alkyloxyalkylene groups. In the formula, the subscript "m" is 0 or 1, "n" is 0, 1, 2, 3, 4 or 5, and R¹⁴ is a fluorine atom or CF₃. Such fluoroalkyloxyfluoroalkylene groups are exemplified by the perfluoroalkyloxyfluoroalkylene groups represented by the formulae presented below: -CH₂CH₂CF(CF₃)-[OCF₂CF(CF₃)]ₙ-OC₃F₇, -CH₂CH₂CF₂CF₂-[OCF₂CF(CF₃)]ₙ-OC₃F₇.

The number-average molecular weight of the vinyl polymer used in the present invention may be between 3000 and 2 000 000 g/mol and more preferably between 5000 and 800 000 g/mol.

This type of fluorinated vinyl polymer can be obtained by addition:
- of a vinyl monomer (M2) without a fluorinated organic group,
- to a vinyl monomer (M1) comprising fluorinated organic groups, and
- a carbosiloxane dendrimer (B) as defined above, of general formula (I) as defined above,
by subjecting them to a copolymerization.

Thus, according to one embodiment, a composition of the invention can comprise a vinyl polymer having at least one unit derived from carbosiloxane dendrimer and resulting from the copolymerization of a vinyl monomer (M1) as defined above, optionally of a vinyl monomer (M2) as defined above, and of a carbosiloxane dendrimer (B) as defined above, said vinyl polymer having a copolymerization ratio of the monomer (M1) to the monomer (M2) of 0.1 to 100:99.9 to 0% by weight, and a copolymerization ratio of the sum of the monomers (M1) and (M2) to the monomer (B) of 0.1 to 99.9:99.9 to 0.1% by weight.

The vinyl monomers (M1) comprising fluorinated organic groups in the molecule are preferably monomers represented by the general formula:

(CH²)=CR¹⁵COOR^{f}.

In this formula, R¹⁵ is a hydrogen atom or a methyl group and R^{f} is a fluorinated organic group exemplified by the fluoroalkyl and fluoroalkyloxyfluoroalkylene groups described above. The compounds represented by the formulae presented below are suggested as specific examples of the component (M1). In the formulae present below, "z" is an integer from 1 to 4.
CH₂=CCH₃COO-CF₃, CH₂=CCH₃COO-C₂F₅, CH₂=CCH3COO-nC₃F₇,
CH₂=CCH₃COO-CF(CF₃)₂, CH₂=CCH₃COO-nC₄F₉,
CH₂=CCH₃COO-CF(CF₃)₂, CH₂=CCH₃COO-nC₅F₁₁,
CH₂=CCH₃COO-nC₆F₁₃, CH₂=CCH₃COO-nC₈F₁₇, CH₂=CCH₃COO-CH₂CF₃,
CH₂=CCH₃COO-CH(CF₃)₂, CH₂=CCH₃COO-CH₂CH(CF₃)₂,
CH₂=CCH₃COO-CH₂(CF₂)₂F, CH₂=CCH₃COO-CH₂(CF₂)₂F,
CH₂=CCH₃COO-CH₂(CF₂)₄F, CH₂=CCH₃COO-CH₂(CF₂)₆F,
CH₂=CCH₃COO-CH₂(CF₂)₈F, CH₂=CCH₃COO-CH₂CH₂CF₃,
CH₂=CCH₃COO-CH₂CH₂(CF₂)₂F, CH₂=CCH₃COO-CH₂CH₂(CF₂)₃F,
CH₂=CCH₃COO-CH₂CH₂(CF₂)₄F, CH₂=CCH₃COO-CH₂CH₂(CF₂)₆F,
CH₂=CCH₃COO-CH₂CH₂(CF₂)₈F, CH₂=CCH₃COO-CH₂CH₂(CF₂)₁₀F,
CH₂=CCH₃COO-CH₂CH₂(CF₂)₁₂F, CH₂=CCH₃COO-CH₂CH₂(CF₂)₁₄F,
CH₂=CCH₃COO-CH₂-CH₂-(CF₂)₁₆F, CH₂=CCH₃COO-CH₂CH₂CH₂CF₃,
CH₂=CCH₃COO-CH₂CH₂CH₂(CF₂)₂F, CH₂=CCH₃COO-CH₂CH₂CH₂(CF₂)₂H,
CH₂=CCH₃COO-CH₂(CF₂)₄H, CH₂=CCH₃COO-(CF₂)₃H,
CH₂=CCH₃COO-CH₂CH₂CF(CF₃)-[OCF₂-CF(CF₃)]z-OC₃F₇,
CH₂=CCH₃COO-CH₂CH₂CF₂CF₂-[OCF₂-CF(CF₃)]z-OC₃F₇,
CH₂=CHCOO-CF₃, CH2=CHCOO-C₂F₅, CH₂=CHCOO-nC₃F₇,
CH₂=CHCOO-CF(CF₃)₂, CH₂=CHCOO-nC₄F₉, CH₂=CHCOO-CF₂CF(CF₃)₂,
CH₂=CHCOO-nC₅F₁₁, CH₂=CHCOO-nC₆F₁₃, CH₂=CHCOO-nC₈F₁₇,
CH₂=CHCOO-CH₂CF₃, CH₂=CHCOO-CH(CF₃)₂, CH₂=CHCOO-CH₂CH(CF₃)₂,
CH₂=CHCOO-CH₂(CF₂)₂F, CH₂=CHCOO-CH₂(CF₂)₃F,
CH₂=CHCOO-CH₂(CF₂)₄F, CH₂=CHCOO-CH₂(CF₂)₆F,
CH₂=CHCOO-CH₂(CF₂)₈F, CH₂=CHCOO-CH₂CH₂CF₃,
CH₂=CHCOO-CH₂CH₂(CF₂)₂F, CH₂=CHCOO-CH₂CH₂(CF₂)₃F,
CH₂=CHCOO-CH₂CH₂(CF₂)₄F, CH₂=CHCOO-CH₂CH₂(CF₂)₆F,
CH₂=CHCOO-CH₂CH₂(CF₂)₈F, CH₂=HCOO-CH₂CH₂(CF₂)₁₀F,
CH₂-CHCOO-CH₂CH₂-(CF₂)₁₂F, CH₂=CHCOO-CH₂CH₂(CF₂)₁₄F,
CH₂=CHCOO-CH₂CH₂-(CF₂)₁₆F, CH₂=CHCOO-CH₂CH₂(CF₂)₃F,
CH₂=CHCOO-CH₂CH₂CH₂(CF₂)₂F, CH₂=CHCOO-CH₂CH₂CH₂(CF)₂H,
CH₂=CHCOO-CH₂(CF₂)₄H, CH₂=CHCOO-CH₂CH₂(CF₂)₃H,
CH₂=CHCOO-CH₂CH₂CF(CF₃)-, [OCF₂-CF(CF₃)]_{Z}-OC₃F₇,
CH₂=CHCOO-CH₂CH₂CF₂CF₂(CF₃)-[OCF₂-CF(CF₃)]₂-OC₃F₇.

Among these, the vinyl polymers represented by the formulae presented below are preferred:
CH₂=CHCOO-CH₂CH₂(CF₂)₆F, CH₂=CHCOO-CH₂CH₂(CF₂)₈F,
CH₂=CCH₃COO-CH₂CH₂(CF₂)₆F, CH₂=CCH₃COO-CH₂CH₂(CF₂)₈F,
CH₂=CHCOO-CH₂CF₃, CH₂=CCH₃COO-CH₂CF₃.

The vinyl polymers represented by the formulae presented below are particularly preferred:
CH₂=CHCOO-CH₂CF₃, CH₂=CCHCOO-CH₂CF₃.

The vinyl monomers (M2) which do not comprise fluorinated organic groups in the molecule can be any monomers having radically polymerizable vinyl groups which are exemplified, for example, by methyl acrylate, methyl methacrylate, ethyl acrylate, ethyl methacrylate, n-propyl acrylate, n-propyl methacrylate, isopropyl acrylate, isopropyl methacrylate and other lower alkyl acrylates or methacrylates; glycidyl acrylate, glycidyl methacrylate; n-butyl acrylate, n-butyl methacrylate, isobutyl acrylate, isobutyl methacrylate, tert-butyl acrylate, tert-butyl methacrylate, n-hexyl acrylate, n-hexyl methacrylate, cyclohexyl acrylate, cyclohexyl methacrylate, 2-ethylhexyl acrylate, 2-ethylhexyl methacrylate, octyl acrylate, octyl methacrylate, lauryl acrylate, lauryl methacrylate, stearyl acrylate, stearyl methacrylate and other higher acrylates and methacrylates; vinyl acetate, vinyl propionate and other lower fatty acid vinyl esters; vinyl butyrate, vinyl caproate, vinyl 2-ethylhexanoate, vinyl laurate, vinyl stearate and other higher fatty acid esters; styrene, vinyltoluene, benzyl acrylate, benzyl methacrylate, phenoxyethyl acrylate, phenoxyethyl methacrylate, vinylpyrrolidone and other vinylaromatic monomers; dimethylaminoethyl acrylate, dimethylaminoethyl methacrylate, diethylaminoethyl acrylate, diethylaminoethyl methacrylate and other aminovinyl monomers, acrylamide, methacrylamide, N-methylolacrylamide, N-methylolmethacrylamide, N-methoxymethylacrylamide, N-methoxymethylmethacrylamide, isobutoxymethoxyacrylamide, isobutoxymethoxymethacrylamide, N,N-dimethylacrylamide, N,N-dimethylmethacrylamide and other vinylamide monomers; hydroxyethyl acrylate, hydroxyethyl methacrylate, acrylic acid hydroxypropyl alcohol, methacrylic acid hydroxypropyl alcohol and other hydroxyvinyl monomers; acrylic acid, methacrylic acid, itaconic acid, crotonic acid, fumaric acid, maleic acid and other vinylcarboxylic acid monomers; tetrahydrofurfuryl acrylate, tetrahydrofurfuryl methacrylate, butoxyethyl acrylate, butoxyethyl methacrylate, ethoxydiethylene glycol acrylate, ethoxydiethylene glycol methacrylate, polyethylene glycol acrylate, polyethylene glycol methacrylate, polypropylene glycol monoacrylate, polypropylene glycol monomethacrylate, hydroxybutyl vinyl ether, cetyl vinyl ether, 2-ethylhexyl vinyl ether and other vinyl monomers having ether bonds; acryloyloxypropyltrimethoxysilane, methacryloyloxypropyltrimethoxysilane, polydimethylsiloxanes comprising acryloyl or methacryloyl groups at one of the ends, polydimethylsiloxanes comprising alkenylaryl groups at one of the ends and other silicone compounds having unsaturated groups; butadiene; vinyl chloride; vinylidene chloride, acrylonitrile, methacrylonitrile; dibutyl fumarate; maleic anhydride; dodecylsuccinic anhydride; acryl glycidyl ether, methacryl glycidyl ether, 3,4-epoxycyclohexylmethyl acrylate, 3,4-epoxycyclohexylmethyl methacrylate, alkali metal salts, ammonium salts and organic amine salts of acrylic acid, of methacrylic acid, of itaconic acid, of crotonic acid, of fumaric acid, of maleic acid and of other radically polymerizable unsaturated carboxylic acids, radically polymerizable unsaturated monomers comprising sulfonic acid groups, such as styrenesulfonic acid, and also their alkali metal salts, their ammonium salts and their organic amine salts; the quaternary ammonium salts resulting from acrylic acid or methacrylic acid, such as 2-hydroxy-3-methacryloyloxypropyltrimethylammonium chloride, methacrylic acid esters of a tertiary amine alcohol, such as the diethylamine ester of methacrylic acid, and their quaternary ammonium salts.

In addition, it is also possible to use, as vinyl monomers (M2), the polyfunctional vinyl monomers which are exemplified, for example, by trimethylolpropane triacrylate, trimethylolpropane trimethacrylate, pentaerythrityl triacrylate, pentaerythrityl trimethacrylate, ethylene glycol diacrylate, ethylene glycol dimethacrylate, tetraethylene glycol diacrylate, tetraethylene glycol dimethacrylate, polyethylene glycol diacrylate, polyethylene glycol dimethacrylate, 1,4-butanediol diacrylate, 1,4-butanediol dimethacrylate, 1,6-hexanediol diacrylate, 1,6-hexanediol dimethacrylate, neopentyl glycol diacrylate, neopentyl glycol dimethacrylate, trimethylolpropane ethoxylate triacrylate, trimethylolpropane ethoxylate trimethacrylate, tris(2-hydroxyethyl)isocyanurate diacrylate, tris(2-hydroxyethyl)isocyanurate dimethacrylate, tris(2-hydroxyethyl)isocyanurate triacrylate, tris(2-hydroxyethyl)isocyanurate trimethacrylate, polydimethylsiloxane, the two ends of the molecular chain of which are blocked with alkenylaryl groups, and other silicone compounds having unsaturated groups.

As regards the abovementioned ratio in which (M1) and (M2) are copolymerized, the ratio by weight of (M1) to (M2) is preferably within the range from 1:99 to 100:0.

Y can be chosen, for example, from organic groups having acrylic or methacrylic groups, organic groups having an alkenylaryl group, or alkenyl groups with from 2 to 10 carbon atoms.

The organic groups having acrylic or methacrylic groups and the alkenylaryl groups are as defined above.

Mention may be made, among the compounds (B), for example, of the following compounds:

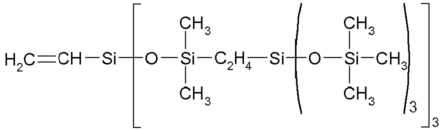

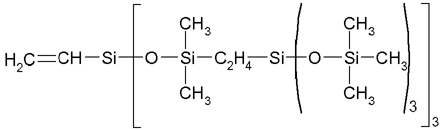

The carbosiloxane dendrimers (B) can be prepared using the process for preparing branched siloxane/silalkylene copolymers described in the document EP 1 055 674.

For example, they can be prepared by subjecting organic alkenyl silicone compounds and silicone compounds comprising hydrogen atoms bonded to the silicon, represented by the formula (IV) as defined above, to a hydrosilylation reaction.

The copolymerization ratio (by weight) of the monomer (B) to the monomers (M1) and (M2) is preferably within the range from 1:99 to 99:1 and even more preferably within the range from 5:95 to 95:5.

Amino groups can be introduced into the side chains of the vinyl polymer using, included in the component (M2), vinyl monomers comprising amino groups, such as dimethylaminoethyl acrylate, dimethylaminoethyl methacrylate, diethylaminoethyl acrylate and diethylaminoethyl methacrylate, followed by performing a modification with potassium acetate monochloride, ammonium acetate monochloride, the aminomethylpropanol salt of monochloroacetic acid, the triethanolamine salt of monobromoacetic acid, sodium monochloropropionate, and other alkali metal salts of halogenated fatty acids; otherwise, carboxylic acid groups can be introduced into the side chains of the vinyl polymer using, included in the component (M2), vinyl monomers comprising carboxylic acids, such as acrylic acid, methacrylic acid, itaconic acid, crotonic acid, fumaric acid and maleic acid, and the like, followed by neutralizing the product with triethylamine, diethylamine, triethanolamine and other amines.

A fluorinated vinyl polymer can be one of the polymers described in the examples of application WO 03/045337.

According to a preferred embodiment, a vinyl polymer grafted in the sense of the present invention may be conveyed in an oil or a mixture of oils, which are preferably volatile, chosen in particular from silicone oils and hydrocarbon-based oils, and mixtures thereof.

According to a particular embodiment, a silicone oil that is suitable for use in the invention may be cyclopentasiloxane.

According to another particular embodiment, a hydrocarbon-based oil that is suitable for use in the invention may be isododecane.

The vinyl polymers grafted with at least one carbosiloxane dendrimer-based unit that may be particularly suitable for the present invention are the polymers of which the INCI name is Acrylates / Polytrimethylsiloxy Methacrylate Copolymer, and which are in particular sold under the names FA 4002 ID Silicone Acrylate and FA 4001 CM Silicone Acrylate, by the company Dow Corning.

According to one embodiment, the composition according to the present invention comprises the vinyl polymer having at least one carbosiloxane dendrimer-based unit in an active material content of from 0.5% to 20%, in particular from 1% to 15%, more particularly from 1.5% to 10% and preferably from 3% to 5% by weight, relative to the weight of said composition.

### SURFACTANTS

According to a particular embodiment of the invention, the composition comprises at least one surfactant.

A surfactant or a mixture of surfactants may be present at from 0.05% to 20% by weight and preferably from 0.5% to 10% by weight, relative to the weight of the composition.

More particularly, the suitable surfactants may be chosen from non-ionic, anionic, cationic and amphoteric surfactants, and mixtures thereof.

For the choice of these surfactants, reference may be made to the document "Encyclopedia of Chemical Technology, Kirk-Othmer", volume 22, pp. 333-432, 3rd edition, 1979, Wiley, for the definition of the properties and functions (emulsifying) of surfactants, in particular pp. 347-377 of this reference, for anionic and non-ionic surfactants.

### Surfactants promoting direct emulsions (oil-in-water; O/W)

Among the suitable surfactants promoting oil-in-water emulsions, mention may be made of the compounds which follow.

### Non-ionic surfactants

In particular, at least one emulsifying surfactant having at 25°C an HLB (hydrophilic-lipophilic balance) within the Griffin sense of greater than or equal to 8 may be used. The HLB value according to Griffin is defined in J. Soc. Cosm. Chem. 1954 (volume 5), pages 249-256.

An emulsifying surfactant having at 25°C an HLB balance (hydrophilic-lipophilic balance) within the Griffin sense of less than 8 may also optionally be used.

The non-ionic surfactants may be chosen especially from alkyl and polyalkyl esters of poly(ethylene oxide), oxyalkylenated alcohols, alkyl and polyalkyl ethers of poly(ethylene oxide), optionally polyoxyethylenated alkyl and polyalkyl esters of sorbitan, optionally polyoxyethylenated alkyl and polyalkyl ethers of sorbitan, alkyl and polyalkyl glycosides or polyglycosides, in particular alkyl and polyalkyl glucosides or polyglucosides, alkyl and polyalkyl esters of sucrose, optionally polyoxyethylenated alkyl and polyalkyl esters of glycerol, and optionally polyoxyethylenated alkyl and polyalkyl ethers of glycerol, and mixtures thereof.
1) Alkyl and polyalkyl esters of polyethylene oxide) that are preferably used are those with a number of ethylene oxide (EO) units ranging from 2 to 200. Examples that may be mentioned include stearate 40 EO, stearate 50 EO, stearate 100 EO, laurate 20 EO, laurate 40 EO and distearate 150 EO.
2) Alkyl and polyalkyl ethers of polyethylene oxide) that are preferably used are those with a number of ethylene oxide (EO) units ranging from 2 to 200. Examples that may be mentioned include cetyl ether 23 EO, oleyl ether 50 EO, phytosterol 30 EO, steareth 40, steareth 100 and beheneth 100.
3) Oxyalkylenated, in particular oxyethylenated and/or oxypropylenated, alcohols that are preferably used are those that can comprise from 1 to 150 oxyethylene and/or oxypropylene units, in particular containing from 20 to 100 oxyethylene units, in particular ethoxylated fatty alcohols, in particular of C₈-C₂₄ and preferably of C₁₂-C₁₈, such as stearyl alcohol ethoxylated with 20 oxyethylene units (CTFA name Steareth-20), for instance Brij 78 sold by the company Uniqema, cetearyl alcohol ethoxylated with 30 oxyethylene units (CTFA name Ceteareth-30), and the mixture of C₁₂-C₁₅ fatty alcohols comprising 7 oxyethylene units (CTFA name C₁₂₋₁₅ Pareth-7), for instance the product sold under the name Neodol 25-7^{®} by Shell Chemicals; or in particular oxyalkylenated (oxyethylenated and/or oxypropylenated) alcohols containing from 1 to 15 oxyethylene and/or oxypropylene units, in particular ethoxylated C₈-C₂₄ and preferably C₁₂-C₁₈ fatty alcohols, such as stearyl alcohol ethoxylated with 2 oxyethylene units (CTFA name Steareth-2), for instance Brij 72 sold by the company Uniqema.
4) Optionally polyoxyethylenated alkyl and polyalkyl esters of sorbitan that are preferably used are those with a number of ethylene oxide (EO) units ranging from 0 to 100. Examples that may be mentioned include sorbitan laurate 4 or 20 EO, in particular polysorbate 20 (or polyoxyethylene (20) sorbitan monolaurate) such as the product Tween 20 sold by the company Uniqema, sorbitan palmitate 20 EO, sorbitan stearate 20 EO, sorbitan oleate 20 EO, or else the Cremophor products (RH 40, RH 60, etc.) from BASF.
5) Optionally polyoxyethylenated alkyl and polyalkyl ethers of sorbitan that are preferably used are those with a number of ethylene oxide (EO) units ranging from 0 to 100.
6) Alkyl and polyalkyl glucosides or polyglucosides that are preferably used are those containing an alkyl group comprising from 6 to 30 carbon atoms and preferably from 6 to 18 or even from 8 to 16 carbon atoms, and containing a glucoside group preferably comprising from 1 to 5 and in particular 1, 2 to 3 glucoside units. The alkylpolyglucosides may be chosen, for example, from decylglucoside (C₉/C₁₁ alkylpolyglucoside (1.4)), for instance the product sold under the name Mydol 10^{®} by the company Kao Chemicals or the product sold under the name Plantacare 2000 UP^{®} by the company Henkel and the product sold under the name Oramix NS 10^{®} by the company SEPPIC; caprylyl/capryl glucoside, for instance the product sold under the name Plantacare KE 3711^{®} by the company Cognis or Oramix CG 110^{®} by the company SEPPIC; laurylglucoside, for instance the product sold under the name Plantacare 1200 UP^{®} by the company Henkel or Plantaren 1200 N^{®} by the company Henkel; cocoglucoside, for instance the product sold under the name Plantacare 818 UP^{®} by the company Henkel; caprylylglucoside, for instance the product sold under the name Plantacare 810 UP^{®} by the company Cognis; and mixtures thereof.
   More generally, the surfactants of alkylpolyglycoside type are defined more specifically hereinbelow.
7) Examples of alkyl and polyalkyl esters of sucrose that may be mentioned are Crodesta F150, sucrose monolaurate sold under the name Crodesta SL 40, and the products sold by Ryoto Sugar Ester, for instance sucrose palmitate sold under the reference Ryoto Sugar Ester P1670, Ryoto Sugar Ester LWA 1695 or Ryoto Sugar Ester 01570.
8) Optionally polyoxyethylenated alkyl and polyalkyl esters of glycerol that are preferably used are those with a number of ethylene oxide (EO) units ranging from 0 to 100 and a number of glycerol units ranging from 1 to 30. Examples that may be mentioned include hexaglyceryl monolaurate and PEG-30 glyceryl stearate.
9) Optionally polyoxyethylenated alkyl and polyalkyl ethers of glycerol that are preferably used are those with a number of ethylene oxide (EO) units ranging from 0 to 100 and a number of glycerol units ranging from 1 to 30. Examples that may be mentioned include Nikkol Batyl Alcohol 100 and Nikkol Chimyl Alcohol 100.

### Anionic surfactants

The anionic surfactants may be chosen from alkyl ether sulfates, carboxylates, amino acid derivatives, sulfonates, isethionates, taurates, sulfosuccinates, alkylsulfoacetates, phosphates and alkyl phosphates, polypeptides, metal salts of C₁₀-C₃₀ and especially C₁₂-C₂₀ fatty acids, in particular metal stearates, and mixtures thereof.
1) Examples of alkyl ether sulfates that may be mentioned include sodium lauryl ether sulfate (70/30 C₁₂₋C₁₄) (2.2 EO) sold under the names Sipon AOS225 or Texapon N702 by the company Henkel, ammonium lauryl ether sulfate (70/30 C₁₂₋C₁₄) (3 EO) sold under the name Sipon LEA 370 by the company Henkel, ammonium (C₁₂-C₁₄) alkyl ether (9 EO) sulfate sold under the name Rhodapex AB/20 by the company Rhodia Chimie, and the mixture of sodium magnesium lauryl oleyl ether sulfate sold under the name Empicol BSD 52 by the company Albright & Wilson.
2) Examples of carboxylates that may be mentioned include salts (for example alkali metal salts) of N-acylamino acids, glycol carboxylates, amido ether carboxylates (AECs) and polyoxyethylenated carboxylic acid salts.

The surfactant of glycol carboxylate type may be chosen from alkyl glycol carboxylics or 2-(2-hydroxyalkyloxyacetate), salts thereof and mixtures thereof. These alkyl glycol carboxylics comprise a linear or branched, saturated or unsaturated, aliphatic and/or aromatic alkyl chain containing from 8 to 18 carbon atoms. These carboxylics may be neutralized with mineral bases such as potassium hydroxide or sodium hydroxide.

Examples of surfactants of glycol carboxylic type that may be mentioned include sodium lauryl glycol carboxylate or sodium 2-(2-hydroxyalkyloxy acetate) such as the product sold under the name Beaulight Shaa^{®} by the company Sanyo, Beaulight LCA-25N^{®} or the corresponding acid form Beaulight Shaa (Acid form)^{®}.

An example of an amido ether carboxylate (AEC) that may be mentioned is sodium lauryl amido ether carboxylate (3 EO) sold under the name Akypo Foam 30^{®} by the company Kao Chemicals.

Examples of polyoxyethylenated carboxylic acid salts that may be mentioned include oxyethylenated (6 EO) sodium lauryl ether carboxylate (65/25/10 C₁₂₋₁₄₋₁₆) sold under the name Akypo Soft 45 NV^{®} by the company Kao Chemicals, polyoxyethylenated and carboxymethylated fatty acids of olive oil origin sold under the name Olivem 400^{®} by the company Biologia e Tecnologia, and oxyethylenated (6 EO) sodium tridecyl ether carboxylate sold under the name Nikkol ECTD-6NEX^{®} by the company Nikkol.

3) Amino acid derivatives that may especially be mentioned include alkaline salts of amino acids, such as:
- sarcosinates, for instance the sodium lauroyl sarcosinate sold under the name Sarkosyl NL 97^{®} by the company Ciba or sold under the name Oramix L30^{®} by the company SEPPIC, sodium myristoyl sarcosinate sold under the name Nikkol Sarcosinate MN^{®} by the company Nikkol, and sodium palmitoyl sarcosinate sold under the name Nikkol Sarcosinate PN^{®} by the company Nikkol;
- alaninates, for instance sodium N-lauroyl N-methyl amidopropionate sold under the name Sodium Nikkol Alaninate LN 30^{®} by the company Nikkol, or sold under the name Alanone ALE^{®} by the company Kawaken, and triethanolamine N-lauroyl N-methyl alanine sold under the name Alanone Alta^{®} by the company Kawaken;
- glutamates, for instance triethanolamine monococoyl glutamate sold under the name Acylglutamate CT-12^{®} by the company Ajinomoto, or triethanolamine lauroyl glutamate sold under the name Acylglutamate LT-12^{®} by the company Ajinomoto.

The glutamic acid salts and/or derivatives are described more specifically hereinbelow.
- aspartates, for instance the mixture of triethanolamine N-lauroyl aspartate and of triethanolamine N-myristoyl aspartate, sold under the name Asparack^{®} by the company Mitsubishi;
- glycine derivatives (glycinates), for instance the sodium N-cocoyl glycinate sold under the names Amilite GCS-12^{®} and Amilite GCK 12 by the company Ajinomoto;
- citrates, such as the oxyethylenated (9 mol) citric monoester of cocoyl alcohols sold under the name Witconol EC 1129 by the company Goldschmidt;
- galacturonates, such as the sodium dodecyl-D-galactoside uronate sold by the company Soliance.

4) Examples of sulfonates that may be mentioned include alpha-olefin sulfonates, for instance the sodium alpha-olefin sulfonate (C₁₄-₁₆) sold under the name Bio-Terge AS-40^{®} by the company Stepan, sold under the names Witconate AOS Protege^{®} and Sulframine AOS PH 12^{®} by the company Witco or sold under the name Bio-Terge AS-40 CG^{®} by the company Stepan, and the sodium secondary olefin sulfonate sold under the name Hostapur SAS 30^{®} by the company Clariant.

5) Isethionates that may be mentioned include acylisethionates, for instance sodium cocoylisethionate, such as the product sold under the name Jordapon Cl P^{®} by the company Jordan.

6) Taurates that may be mentioned include the sodium salt of palm kernel oil methyltaurate sold under the name Hostapon CT Pate^{®} by the company Clariant; N-acyl N-methyltaurates, for instance the sodium N-cocoyl N-methyltaurate sold under the name Hostapon LT-SF^{®} by the company Clariant or sold under the name Nikkol CMT-30-T^{®} by the company Nikkol, and the sodium palmitoyl methyltaurate sold under the name Nikkol PMT^{®} by the company Nikkol.

7) Examples of sulfosuccinates that may be mentioned include the oxyethylenated (3 EO) lauryl alcohol monosulfosuccinate (70/30 C₁₂/C₁₄) sold under the names Setacin 103 Special^{®} and Rewopol SB-FA 30 K 4^{®} by the company Witco, the disodium salt of a C₁₂-C₁₄ alcohol hemisulfosuccinate, sold under the name Setacin F Special Paste^{®} by the company Zschimmer Schwarz, the oxyethylenated (2 EO) disodium oleamidosulfosuccinate sold under the name Standapol SH 135^{®} by the company Henkel, the oxyethylenated (5 EO) laurylamide monosulfosuccinate sold under the name Lebon A-5000^{®} by the company Sanyo, the oxyethylenated (10 EO) disodium salt of lauryl citrate monosulfosuccinate sold under the name Rewopol SB CS 50^{®} by the company Witco, and the ricinoleic monoethanolamide monosulfosuccinate sold under the name Rewoderm S 1333^{®} by the company Witco. Polydimethylsiloxane sulfosuccinates may also be used, such as disodium PEG-12 dimethicone sulfosuccinate sold under the name Mackanate-DC30 by the company Maclntyre.

8) Examples of alkyl sulfoacetates that may be mentioned include the mixture of sodium lauryl sulfoacetate and disodium lauryl ether sulfosuccinate, sold under the name Stepan-Mild LSB by the company Stepan.

9) Examples of phosphates and alkyl phosphates that may be mentioned include monoalkyl phosphates and dialkyl phosphates, such as the lauryl monophosphate sold under the name MAP 20^{®} by the company Kao Chemicals, the potassium salt of dodecylphosphoric acid, mixture of monoester and diester (predominantly diester), sold under the name Crafol AP-31^{®} by the company Cognis, the mixture of octylphosphoric acid monoester and diester sold under the name Crafol AP-20^{®} by the company Cognis, the mixture of ethoxylated (7 mol of EO) phosphoric acid monoester and diester of 2-butyloctanol, sold under the name Isofol 12 7 EO-Phosphate Ester^{®} by the company Condea, the potassium or triethanolamine salt of mono(C₁₂-C₁₃)alkyl phosphate sold under the references Arlatone MAP 230K-40^{®} and Arlatone MAP 230T-60^{®} by the company Uniqema, the potassium lauryl phosphate sold under the name Dermalcare MAP XC-99/09^{®} by the company Rhodia Chimie, and the potassium cetyl phosphate sold under the name Arlatone MAP 160K by the company Uniqema.

10) The polypeptides are obtained, for example, by condensation of a fatty chain onto amino acids from cereal and in particular from wheat and oat. Examples of polypeptides that may be mentioned include the potassium salt of hydrolysed lauroyl wheat protein, sold under the name Aminofoam W OR by the company Croda, the triethanolamine salt of hydrolysed cocoyl soybean protein, sold under the name May-Tein SY by the company Maybrook, the sodium salt of lauroyl oat amino acids, sold under the name Proteol Oat by the company SEPPIC, collagen hydrolysate grafted onto coconut fatty acid, sold under the name Geliderm 3000 by the company Deutsche Gelatine, and soybean proteins acylated with hydrogenated coconut acids, sold under the name Proteol VS 22 by the company SEPPIC.

11) As metal salts of C₁₀-C₃₀ and especially C₁₂-C₂₀ fatty acids, mention may be made in particular of metal stearates, such as sodium stearate and potassium stearate, and also polyhydroxystearates.

### Cationic surfactants

The cationic surfactants may be chosen from:
- alkylimidazolidiniums such as isostearylethylimidonium ethosulfate,
- ammonium salts, such as (C₁₂₋₃₀ alkyl)tri(C₁₋₄ alkyl)
ammonium halides, for instance N,N,N-trimethyl-1-docosanaminium chloride (or behentrimonium chloride).

### Amphoteric surfactants

The compositions according to the invention may also contain one or more amphoteric surfactants, for instance N-acylamino acids such as N-alkyl aminoacetates and disodium cocoamphodiacetate, and amine oxides such as stearamine oxide, or alternatively silicone surfactants, for instance dimethicone copolyol phosphates such as the product sold under the name Pecosil PS 100^{®} by the company Phoenix Chemical.

### Silicone surfactants

According to a second embodiment, the composition comprises at least one silicone surfactant. Examples that may be mentioned include:
a) as non-ionic surfactants with an HLB of greater than or equal to 8 at 25°C, used alone or as a mixture; mention may be made in particular of:
   - dimethicone copolyol, such as the product sold under the name Q2-5220^{®} by the company Dow Corning;
   - dimethicone copolyol benzoate, such as the product sold under the names Finsolv SLB 101^{®} and 201^{®} by the company Fintex;
b) as non-ionic surfactants with an HLB of less than 8 at 25°C, used alone or as a mixture, mention may be made in particular of:
   - the mixture of cyclomethicone/dimethicone copolyol sold under the name Q2-3225C^{®} by the company Dow Corning.

### Surfactants promoting inverse emulsions (water-in-oil; W/O)

As emulsifying surfactants that may be used for the preparation of the W/O emulsions, examples that may be mentioned include sorbitan alkyl esters or ethers; silicone surfactants, for instance dimethicone copolyols, such as the mixture of cyclomethicone and of dimethicone copolyol, sold under the name DC 5225 C by the company Dow Corning, and alkyldimethicone copolyols such as laurylmethicone copolyol sold under the name Dow Corning 5200 Formulation Aid by the company Dow Corning; cetyldimethicone copolyol, such as the product sold under the name Abil EM 90R by the company Goldschmidt, and the mixture of cetyldimethicone copolyol, of polyglyceryl isostearate (4 mol) and of hexyl laurate, sold under the name Abil WE O9 by the company Goldschmidt, or else phosphated surfactants.

One or more coemulsifiers, which may be chosen advantageously from the group comprising polyol alkyl esters, may also be added thereto.

Polyol alkyl esters that may in particular be mentioned include polyethylene glycol esters, for instance PEG-30 dipolyhydroxystearate, such as the product sold under the name Arlacel P135 by the company ICI.

A crosslinked elastomeric solid organopolysiloxane comprising at least one oxyalkylene group, such as the products obtained according to the procedure of Examples 3, 4 and 8 of document US-A-5 412 004 and the examples of document US-A-5 811 487, in particular the product of Example 3 (synthetic example) of patent US-A-5 412 004, and such as the product sold under the reference KSG 21 by the company Shin-Etsu, may also be used as surfactants for W/O emulsions.

According to one particularly preferred embodiment, an emulsion according to the invention, in particular a W/O emulsion, comprises at least one silicone surfactant, more particularly chosen from dimethicone copolyols.

A dimethicone copolyol that may be used according to the invention is an oxypropylenated and/or oxyethylenated polydimethylmethylsiloxane.

Dimethicone copolyols that may be used are those corresponding more particularly to formula (II) below: 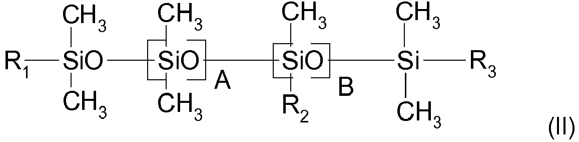 in which:
- R₁, R₂ and R₃, independently of each other, represent a C₁-C₆ alkyl radical or a radical -(CH₂)ₓ-(OCH₂CH₂)_{y}-(OCH₂CH₂CH₂)_{z}-OR₄, at least one radical R₁, R₂ or R₃ not being an alkyl radical; R₄ being a hydrogen, a C₁-C₃ alkyl radical or a C₂-C₄ acyl radical;
- A is an integer ranging from 0 to 200;
- B is an integer ranging from 0 to 50; on condition that A and B are not simultaneously equal to zero;
- x is an integer ranging from 1 to 6;
- y is an integer ranging from 1 to 30; and
- z is an integer ranging from 0 to 30, preferably from 0 to 20.

According to one preferred embodiment, in the compound of formula (II), R₁ = R₃ = methyl radical, x is an integer ranging from 2 to 6 and y is an integer ranging from 4 to 30. R₄ is in particular a hydrogen.

Examples of compounds of formula (II) that may be mentioned include the compounds of formula (III): 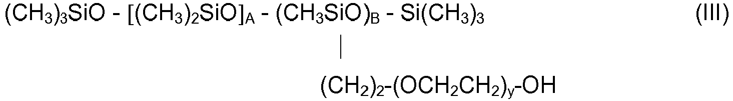 in which A is an integer ranging from 20 to 105, B is an integer ranging from 2 to 10 and y is an integer ranging from 10 to 20.

Examples of silicone compounds of formula (II) that may also be mentioned include the compounds of formula (IV):

HO - (OCH₂CH₂)_{y}-(CH₂)₃ - [(CH₃)₂SiO]_{A'} - (CH₂)₃ - (OCH₂CH₂)_{y} - OH (IV)

in which A' and y are integers ranging from 10 to 20.

Dimethicone copolyols that may be used include those sold under the names DC 5329, DC 7439-146, DC 2-5695 and Q4-3667 by the company Dow Corning; KF-6013, KF-6015, KF-6016, KF-6017, KF-6028 and KF-6050 L, by the company Shin-Etsu.

The compounds DC 5329, DC 7439-146 and DC 2-5695 are compounds of formula (III) in which, respectively, A is 22, B is 2 and y is 12; A is 103, B is 10 and y is 12; A is 27, B is 3 and y is 12.

According to a particular embodiment, the silicone surfactant may be PEG polydimethylsiloxyethyl dimethicone, sold in particular by the company Shin-Etsu under the reference KF-6028, PEG-10 dimethicone sold in particular by the company Shin-Etsu under the reference KF-6017, and mixtures thereof.

The surfactant may also be chosen from non-ionic surfactants of the type of monoglycerolated or polyglycerolated fatty alcohols which can be represented by formula (V) below: 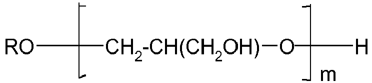 in which:
R represents a linear or branched, saturated or unsaturated radical comprising from 8 to 40 carbon atoms and preferably from 10 to 30 carbon atoms;
m represents a number ranging from 1 to 10.

As compounds of this type, mention may be made of lauryl alcohol comprising 4 mol of glycerol, isostearyl alcohol comprising 4 mol of glycerol, lauryl alcohol comprising 1.5 mol of glycerol, oleyl alcohol comprising 4 mol of glycerol, oleyl alcohol comprising 2 mol of glycerol, cetearyl alcohol comprising 2 mol of glycerol, cetearyl alcohol comprising 6 mol of glycerol, oleocetyl alcohol comprising 6 mol of glycerol, and octadecanol comprising 6 mol of glycerol.

The fatty alcohol can represent a mixture of fatty alcohols in the same way that the value of m represents a statistical value, which means that, in a commercial product, several types of polyglycerolated fatty alcohols can coexist in the form of a mixture.

Preferably, whatever the direction of the emulsion, the surfactant(s) is (are) chosen from non-ionic surfactants and silicone surfactants, or mixtures thereof.

### VOLATILE OILS

According to one particular embodiment of the invention, the composition comprises at least one volatile oil.

The volatile oil(s) may be chosen from hydrocarbon-based oils, silicone oils and fluorinated oils, and mixtures thereof.

The volatile hydrocarbon-based oils are preferably chosen from non-polar hydrocarbon-based oils and may in particular be chosen from volatile hydrocarbon-based oils having from 8 to 16 carbon atoms and mixtures thereof, and in particular:
- branched C₈-C₁₆ alkanes such as C₈-C₁₆ isoalkanes (also known as isoparaffins), isododecane, isodecane and isohexadecane, and, for example, the oils sold under the trade name Isopar or Permethyl,
- linear alkanes, for instance n-dodecane (C₁₂) and n-tetradecane (C₁₄) sold by Sasol under the respective references Parafol 12-97 and Parafol 14-97, and also mixtures thereof, the undecane-tridecane mixture (Cétiol UT), the mixtures of n-undecane (C₁₁) and of n-tridecane (C₁₃) obtained in Examples 1 and 2 of patent application WO 2008/155 059 from the company Cognis, and
- mixtures thereof.

The volatile silicone oils can be chosen from silicone oils with a flash point ranging from 40°C to 102°C, preferably with a flash point of greater than 55°C and less than or equal to 95°C, and preferentially ranging from 65°C to 95°C.

As volatile silicone oils that may be used in the invention, mention may be made of linear or cyclic silicones with a viscosity at 25°C of less than 8 centistokes (cSt) (8 × 10⁻⁶ m²/s), and in particular containing from 2 to 10 silicon atoms and in particular from 2 to 7 silicon atoms, these silicones optionally comprising alkyl or alkoxy groups containing from 1 to 10 carbon atoms.

As volatile silicone oil(s) that may be used in the invention, mention may be made especially of dimethicones with viscosities of 2, 5 and 6 cSt, octamethylcyclotetrasiloxane, decamethylcyclopentasiloxane, dodecamethylcyclohexa-siloxane, heptamethylhexyltrisiloxane, heptamethyloctyltrisiloxane, hexamethyldi-siloxane, octamethyltrisiloxane, decamethyltetrasiloxane and dodecamethylpenta-siloxane, and mixtures thereof.

The volatile oils can also be chosen from a fluorinated oil, such as nonafluoromethoxybutane or perfluoromethylcyclopentane, and mixtures thereof.

Preferably, the composition according to the invention comprises at least one volatile oil, more particularly chosen from non-polar volatile hydrocarbon-based oils, from volatile silicone oils, alone or as mixtures.

According to one particular embodiment, the composition comprises at least one volatile oil in a content ranging from 5% to 45% by weight, in particular from 10% to 30% by weight, relative to the weight of said composition.

### AQUEOUS PHASE

The composition according to the invention comprises water and optionally at least one water-soluble solvent (the whole constituting the aqueous phase).

For the purposes of the present invention, the term "water-soluble solvent" is intended to mean a compound that is liquid at 25°C and atmospheric pressure, and that is water-miscible (miscibility with water of greater than 50% by weight at 25°C and atmospheric pressure).

Among the water-soluble solvents that may be used in the compositions in accordance with the invention, mention may be made in particular of monoalcohols containing from 1 to 5 carbon atoms, such as preferably ethanol and isopropanol, glycols containing from 2 to 8 carbon atoms, such as ethylene glycol, propylene glycol, 1,3-butylene glycol and dipropylene glycol, C₃ and C₄ ketones and C₂-C₄ aldehydes.

The composition according to the invention preferentially comprises at least 20% by weight of aqueous phase (water+water-soluble solvent(s)), in particular from 20% to 60% by weight and especially from 25% to 50% by weight, relative to the weight of the composition.

The composition according to the invention preferentially comprises at least 15% by weight, in particular from 20% to 60% by weight and especially from 25% to 50% by weight of water, relative to the weight of the composition.

According to one embodiment, the composition may comprise at least 60% by weight, preferably at least 70% by weight and in particular at least 75% by weight of water, relative to the total weight of the aqueous phase.

According to a preferred embodiment, the composition comprises a total content of aqueous phase and volatile oil(s) of greater than or equal to 50% by weight and in particular greater than or equal to 60% by weight relative to the weight of the composition.

### DYESTUFFS:

A composition in accordance with the present invention may comprise at least one dyestuff, which may be chosen from water-soluble or water-insoluble, liposoluble or non-liposoluble, organic or inorganic dyestuffs, and materials with an optical effect, and mixtures thereof.

For the purposes of the present invention, the term "dyestuff" is intended to mean a compound that is capable of producing a coloured optical effect when it is formulated in a sufficient amount in a suitable cosmetic medium.

The water-soluble colorants used according to the invention are more particularly water-soluble dyes.

For the purposes of the invention, the term "water-soluble dye" is intended to mean any natural or synthetic, generally organic compound, which is soluble in an aqueous phase or water-miscible solvents and which is capable of colouring. In particular, the term "water-soluble" is intended to characterize the capacity of a compound to be dissolved in water, measured at 25°C, to a concentration at least equal to 0.1 g/l (production of a macroscopically isotropic, transparent, coloured or colourless solution). This solubility is in particular greater than or equal to 1 g/l.

As water-soluble dyes that are suitable for use in the invention, mention may be made in particular of synthetic or natural water-soluble dyes, for instance FDC Red 4 (Cl: 14700), DC Red 6 (Lithol Rubine Na; CI: 15850), DC Red 22 (Cl: 45380), DC Red 28 (Cl: 45410 Na salt), DC Red 30 (Cl: 73360), DC Red 33 (Cl: 17200), DC Orange 4 (Cl: 15510), FDC Yellow 5 (Cl: 19140), FDC Yellow 6 (Cl: 15985), DC Yellow 8 (Cl: 45350 Na salt), FDC Green 3 (Cl: 42053), DC Green 5 (Cl: 61570), FDC Blue 1 (Cl: 42090).

As non-limiting illustrations of sources of water-soluble dyestuff(s) that may be used in the context of the present invention, mention may be made especially of those of natural origin, such as extracts of carmine of cochineal, of beetroot, of grape, of carrot, of tomato, of annatto, of paprika, of henna, of caramel and of curcumin.

Thus, the water-soluble dyestuffs that are suitable for use in the invention are especially carminic acid, betanin, anthocyans, enocyanins, lycopene, beta-carotene, bixin, norbixin, capsanthin, capsorubin, flavoxanthin, lutein, cryptoxanthin, rubixanthin, violaxanthin, riboflavin, rhodoxanthin, cantaxanthin and chlorophyll, and mixtures thereof.

They may also be copper sulfate, iron sulfate, water-soluble sulfopolyesters, rhodamine, betaine, methylene blue, the disodium salt of tartrazine and the disodium salt of fuchsin.

Some of these water-soluble dyestuffs are in particular permitted for food use. Representatives of these dyes that may be mentioned more particularly include dyes of the carotenoid family, referenced under the food codes E120, E162, E163, E160a-g, E150a, E101, E100, E140 and E141.

According to a particularly preferred embodiment, the water-soluble dyestuff(s) are chosen from the disodium salt of brilliant yellow FCF sold by the company LCW under the name DC Yellow 6, the disodium salt of fuchsin acid D sold by the company LCW under the name DC Red 33, and the trisodium salt of Rouge Allura sold by the company LCW under the name FD & C Red 40.

The term "pigments" should be understood as meaning white or coloured, inorganic (mineral) or organic particles, which are insoluble in the liquid organic phase, and which are intended to colour and/or opacify the composition and/or the deposit produced with the composition.

The pigments may be chosen from mineral pigments, organic pigments and composite pigments (i.e. pigments based on mineral and/or organic materials).

The pigments may be chosen from monochromatic pigments, lakes, nacres, and pigments with an optical effect, for instance reflective pigments and goniochromatic pigments.

The mineral pigments may be chosen from metal oxide pigments, chromium oxides, iron oxides, titanium dioxide, zinc oxides, cerium oxides, zirconium oxides, manganese violet, Prussian blue, ultramarine blue and ferric blue, and mixtures thereof.

Organic lakes are organic pigments formed from a dye attached to a substrate.

The lakes, which are also known as organic pigments, may be chosen from the materials below, and mixtures thereof:
- cochineal carmine;
- organic pigments of azo dyes, anthraquinone dyes, indigoid dyes, xanthene dyes, pyrene dyes, quinoline dyes, triphenylmethane dyes or fluoran dyes. Mention may in particular be made, among the organic pigments, of those known under the following names: D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5 or FD&C Yellow No. 6;
- the organic lakes can be insoluble sodium, potassium, calcium, barium, aluminium, zirconium, strontium or titanium salts of acid dyes, such as azo, anthraquinone, indigoid, xanthene, pyrene, quinoline, triphenylmethane or fluoran dyes, these dyes possibly comprising at least one carboxylic or sulfonic acid group.

The organic lakes can also be supported by an organic support, such as rosin or aluminium benzoate, for example.

Mention may in particular be made, among the organic lakes, of those known under the following names: D&C Red No. 2 Aluminium lake, D&C Red No. 3 Aluminium lake, D&C Red No. 4 Aluminium lake, D&C Red No. 6 Aluminium lake, D&C Red No. 6 Barium lake, D&C Red No. 6 Barium/Strontium lake, D&C Red No. 6 Strontium lake, D&C Red No. 6 Potassium lake, D&C Red No. 7 Aluminium lake, D&C Red No. 7 Barium lake, D&C Red No. 7 Calcium lake, D&C Red No. 7 Calcium/Strontium lake, D&C Red No. 7 Zirconium lake, D&C Red No. 8 Sodium lake, D&C Red No. 9 Aluminium lake, D&C Red No. 9 Barium lake, D&C Red No. 9 Barium/Strontium lake, D&C Red No. 9 Zirconium lake, D&C Red No. 10 Sodium lake, D&C Red No. 19 Aluminium lake, D&C Red No. 19 Barium lake, D&C Red No. 19 Zirconium lake, D&C Red No. 21 Aluminium lake, D&C Red No. 21 Zirconium lake, D&C Red No. 22 Aluminium lake, D&C Red No. 27 Aluminium lake, D&C Red No. 27 Aluminium/Titanium/Zirconium lake, D&C Red No. 27 Barium lake, D&C Red No. 27 Calcium lake, D&C Red No. 27 Zirconium lake, D&C Red No. 28 Aluminium lake, D&C Red No. 30 lake, D&C Red No. 31 Calcium lake, D&C Red No. 33 Aluminium lake, D&C Red No. 34 Calcium lake, D&C Red No. 36 lake, D&C Red No. 40 Aluminium lake, D&C Blue No. 1 Aluminium lake, D&C Green No. 3 Aluminium lake, D&C Orange No. 4 Aluminium lake, D&C Orange No. 5 Aluminium lake, D&C Orange No. 5 Zirconium lake, D&C Orange No. 10 Aluminium lake, D&C Orange No. 17 Barium lake, D&C Yellow No. 5 Aluminium lake, D&C Yellow No. 5 Zirconium lake, D&C Yellow No. 6 Aluminium lake, D&C Yellow No. 7 Zirconium lake, D&C Yellow No. 10 Aluminium lake, FD&C Blue No. 1 Aluminium lake, FD&C Red No. 4 Aluminium lake, FD&C Red No. 40 Aluminium lake, FD&C Yellow No. 5 Aluminium lake or FD&C Yellow No. 6 Aluminium lake.

Mention may also be made of liposoluble dyes, such as, for example, Sudan Red, DC Red 17, DC Green 6, β-carotene, soybean oil, Sudan Brown, DC Yellow 11, DC Violet 2, DC Orange 5 or quinoline yellow.

The chemical substances corresponding to each of the organic colorants cited above are mentioned in the publication "International Cosmetic Ingredient Dictionary and Handbook", 1997 edition, pages 371 to 386 and 524 to 528, published by The Cosmetic, Toiletry, and Fragrance Association.

The pigments may also have been subjected to a hydrophobic treatment.

The hydrophobic treatment agent may be chosen from silicones such as methicones, dimethicones and perfluoroalkylsilanes; fatty acids such as stearic acid; metal soaps such as aluminium dimyristate, the aluminium salt of hydrogenated tallow glutamate, perfluoroalkyl phosphates, perfluoroalkylsilanes, perfluoroalkylsilazanes, polyhexafluoropropylene oxides, polyorganosiloxanes comprising perfluoroalkyl perfluoropolyether groups, amino acids; N-acylated amino acids or salts thereof; lecithin, isopropyl triisostearyl titanate, and mixtures thereof.

The N-acylated amino acids can comprise an acyl group having from 8 to 22 carbon atoms, such as, for example, a 2-ethylhexanoyl, caproyl, lauroyl, myristoyl, palmitoyl, stearoyl or cocoyl group. The salts of these compounds can be aluminium, magnesium, calcium, zirconium, zinc, sodium or potassium salts. The amino acid can, for example, be lysine, glutamic acid or alanine.

The term "alkyl" cited in the abovementioned compounds denotes in particular an alkyl group having from 1 to 30 carbon atoms and preferably having from 5 to 16 carbon atoms.

Hydrophobic treated pigments are described in particular in application EP-A-1 086 683.

Within the meaning of the present patent application, "nacre" is intended to mean coloured particles of any shape, which are or are not iridescent, produced in particular by certain molluscs in their shells or else synthesized, and which exhibit a colour effect via optical interference.

Mention may be made, as examples of nacres, of pearlescent pigments, such as titanium mica covered with an iron oxide, mica covered with bismuth oxychloride, titanium mica covered with chromium oxide, titanium mica covered with an organic dye, in particular of the abovementioned type, and also pearlescent pigments based on bismuth oxychloride. They may also be mica particles, at the surface of which are superposed at least two successive layers of metal oxides and/or of organic colorants.

The nacres may more particularly have a yellow, pink, red, bronze, orangey, brown, gold and/or coppery colour or glint.

As illustrations of nacres that may be introduced as interference pigments into the first composition, mention may be made of the gold-coloured nacres sold in particular by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold in particular by the company Merck under the name Bronze fine (17384) (Colorona) and Bronze (17353) (Colorona) and by the company Engelhard under the name Super bronze (Cloisonne); the orange nacres sold in particular by the company Engelhard under the name Orange 363C (Cloisonne) and Orange MCR 101 (Cosmica) and by the company Merck under the name Passion orange (Colorona) and Matte orange (17449) (Microna); the brown nacres sold in particular by the company Engelhard under the name Nu-antique copper 340XB (Cloisonne) and Brown CL4509 (Chromalite); the nacres with a copper glint sold in particular by the company Engelhard under the name Copper 340A (Timica); the nacres with a red glint sold in particular by the company Merck under the name Sienna fine (17386) (Colorona); the nacres with a yellow glint sold in particular by the company Engelhard under the name Yellow (4502) (Chromalite); the red nacres with a gold glint sold in particular by the company Engelhard under the name Sunstone G012 (Gemtone); the pink nacres sold in particular by the company Engelhard under the name Tan opale G005 (Gemtone); the black nacres with a gold glint sold in particular by the company Engelhard under the name Nu antique bronze 240 AB (Timica), the blue nacres sold in particular by the company Merck under the name Matte blue (17433) (Microna), the white nacres with a silvery glint sold in particular by the company Merck under the name Xirona Silver, and the golden-green pink-orange nacres sold in particular by the company Merck under the name Indian summer (Xirona), and mixtures thereof.

### ADDITIVES

The composition according to the invention may also comprise any additive chosen by those skilled in the art such that the advantageous properties intrinsically associated with the compositions in accordance with the invention are not, or not substantially, adversely affected by the envisaged addition(s).

As additives that may be incorporated into the compositions in accordance with the invention, mention may be made especially of hydrophilic thickeners, hydrophobic thickeners, fillers of organic or mineral nature, stabilizers, preserving agents, sweeteners, cosmetic active agents, flavourings or fragrances, pigment dispersers, and film-forming agents other than those according to the invention.

As cosmetic active agents, mention may be made of sunscreens, vitamins A, E, C and B3, provitamins such as D-panthenol, calmatives such as α-bisabolol, Aloe vera, allantoin, plant extracts or essential oils, protective or restructuring agents, refreshing agents such as menthol and derivatives thereof, emollients, moisturizers, antiwrinkle active agents and essential fatty acids, and mixtures thereof.

As examples of hydrophilic thickening polymers, mention may be made more particularly of:
- homopolymers or copolymers of acrylic acid or methacrylic acid, or salts thereof and esters thereof, and in particular the products sold under the names Versicol F or Versicol K by the company Allied Colloid, Ultrahold 8 by the company Ciba-Geigy, and polyacrylic acids of Synthalen K type, and salts, in particular sodium salts, of polyacrylic acid (corresponding to the INCI name sodium acrylate copolymer) and more particularly a crosslinked sodium polyacrylate (corresponding to the INCI name sodium acrylate copolymer (and) caprylic/capric triglyceride) sold under the name Luvigel EM by the company BASF,
- copolymers of acrylic acid and of acrylamide sold in the form of the sodium salt thereof under the name Reten by the company Hercules, the sodium polymethacrylate sold under the name Darvan No. 7 by the company Vanderbilt, and the sodium salts of polyhydroxycarboxylic acids sold under the name Hydagen F by the company Henkel,

- polyacrylic acid/alkyl acrylate copolymers, preferably modified or unmodified carboxyvinyl polymers, particularly with acrylate/C₁₀-C₃₀-alkylacrylate copolymers (INCI name: Acrylates/C₁₀₋₃₀ Alkyl acrylate Crosspolymer) such as the products sold by the company Lubrizol under the trade names Pemulen TR1, Pemulen TR2, Carbopol 1382 and Carbopol EDT 2020, and even more preferentially Pemulen TR-2,
- polyacrylamidomethylpropanesulfonic acid partially neutralized with aqueous ammonia and highly crosslinked, sold by the company Clariant,
- acrylamidopropanesulfonic/acrylamide copolymers of Sepigel or Simulgel type sold by the company SEPPIC,
- polyoxyethylenated acrylamidopropanesulfonic/alkyl methacrylate copolymers (crosslinked or non-crosslinked) of the Aristoflex HMS type sold by the company Clariant,
- copolymers of hydroxyalkylacrylic acid or salts thereof and of acryloyldimethyl taurate monomers such as the product Sepinov EMT 10 sold by the company SEPPIC,
- and mixtures thereof.

Other examples of hydrophilic gelling polymers that may be mentioned include:
- anionic, cationic, amphoteric or non-ionic chitin or chitosan polymers;
- cellulose polymers, for instance alkylcelluloses such as hydroxyethylcellulose, hydroxypropylcellulose, hydroxymethylcellulose, ethylhydroxyethylcellulose and carboxymethylcellulose, and also quaternized cellulose derivatives;
- vinyl polymers, for instance polyvinylpyrrolidones, copolymers of methyl vinyl ether and of malic anhydride, the copolymer of vinyl acetate and of crotonic acid, copolymers of vinylpyrrolidone and of vinyl acetate; copolymers of vinylpyrrolidone and of caprolactam; polyvinyl alcohol;
- optionally modified polymers of natural origin, such as galactomannans and derivatives thereof, for instance konjac gum, gellan gum, locust bean gum, fenugreek gum, karaya gum, gum tragacanth, gum arabic, acacia gum, guar gum, hydroxypropyl guar, hydroxypropyl guar modified with sodium methylcarboxylate groups (Jaguar XC97-1, Rhodia), hydroxypropyltrimethylammonium guar chloride, and xanthan gum and derivatives thereof;
- alginates and carrageenans;
- muccopolysaccharides such as hyaluronic acid;
- and mixtures thereof.

If the composition comprises any, the content of hydrophilic thickener ranges from 0.01% to 3% by weight, preferably from 0.05% to 2% by weight and more advantageously from 0.1% to 1% by weight relative to the weight of the composition.

By way of hydrophobic thickeners, mention may most particularly be made of hydrophobic mineral thickeners such as modified clays, modified silicas, or mixtures thereof.

### Hydrophobic modified clays

Clays are silicates containing a cation which can be chosen from calcium, magnesium, aluminium, sodium, potassium or lithium cations, and mixtures thereof.

Mention may be made, as examples of such products, of clays of the family of the smectites, and also of the family of the vermiculites, stevensite or chlorites. These clays may be of natural or synthetic origin.

Preferably, use is made of organophilic clays, more particularly of modified clays, such as montmorillonite, bentonite, hectorite, attapulgite or sepiolite, and mixtures thereof. The clay is preferably a bentonite or a hectorite.

These clays are modified with a chemical compound chosen from quaternary amines, tertiary amines, amine acetates, imidazolines, amine soaps, fatty sulfates, alkylarylsulfonates or amine oxides, and mixtures thereof.

Mention may thus be made of hectorites modified by a quaternary amine, more specifically by a C₁₀ to C₂₂ fatty acid ammonium halide, such as chloride, comprising or not comprising an aromatic group, such as hectorite modified by a distearyldimethylammonium halide, preferably chloride (CTFA name: Disteardimonium hectorite), such as, for example, that sold under the name Bentone 38V, Bentone 38V CG or Bentone EW CE by Elementis, or stearalkonium hectorites, such as in particular the product Bentone 27 V.

Mention may also be made of quaternium-18 bentonites, such as those sold, inter alia, under the names Bentone 34 by the company Elementis, Claytone 40, Tixogel VP by the company United Catalyst by the company Southern Clay; stearalkonium bentonites, such as those sold under the names Tixogel LG by the company United Catalyst and Claytone AF and Claytone APA by the company Southern Clay; or quaternium-18/benzalkonium bentonites, such as those sold under the name Claytone HT by the company Southern Clay.

According to a preferred embodiment, the thickening agent is chosen from organophilic modified clays, in particular organophilic modified hectorites, in particular modified by benzyldimethylammonium stearate or distearyldimethylammonium halides, in particular chlorides.

### Modified silicas

Mention may also be made of fumed silica hydrophobically treated at the surface, the size of the particles of which is advantageously less than 1 µm. This is because it is possible to chemically modify the surface of the silica, by chemical reaction generating a reduction in the number of silanol groups present at the surface of the silica. It is especially possible to substitute silanol groups with hydrophobic groups: a hydrophobic silica is then obtained. The hydrophobic groups may be:
- trimethylsiloxyl groups, which are obtained especially by treating fumed silica in the presence of hexamethyldisilazane. Silicas thus treated are known as Silica Silylate according to the CTFA (6th edition, 1995). They are sold, for example, under the references Aerosil R812^{®} by the company Degussa and Cab-O-Sil TS-530^{®} by the company Cabot,
- dimethylsilyloxyl or polydimethylsiloxane groups, which are obtained especially by treating fumed silica in the presence of polydimethylsiloxane, for example hexamethyldisiloxane, or dimethyldichlorosilane. Silicas thus treated are known as Silica Dimethyl Silylate according to the CTFA (6th edition, 1995). They are sold, for example, under the references Aerosil R972^{®} and Aerosil R974^{®} by the company Degussa and Cab-O-Sil TS-610^{®} and Cab-O-Sil TS-720^{®} by the company Cabot.

The hydrophobic fumed silica in particular has a particle size that may be nanometric to micrometric, for example ranging from about 5 to 200 nm.

The composition according to the invention can also comprise at least silica aerogel particles.

Silica aerogels are porous materials obtained by replacing (by drying) the liquid component of a silica gel with air.

They are generally synthesized via a sol-gel process in a liquid medium and then dried, usually by extraction with a supercritical fluid, the one most commonly used being supercritical CO₂. Drying of this type makes it possible to avoid contraction of the pores and of the material. The sol-gel process and the various drying operations are described in detail in Brinker C.J. and Scherer G.W., Sol-Gel Science, New York, Academic Press, 1990.

The hydrophobic silica aerogel particles suitable for the implementation of the invention exhibit a specific surface area per unit mass (S_{M}) ranging from 500 to 1500 m²/g, preferably from 600 to 1200 m²/g and better still from 600 to 800 m²/g, and a size, expressed as the volume-average diameter (D[0.5]), ranging from 1 to 1500 µm, better still from 1 to 1000 µm, preferably from 1 to 100 µm, in particular from 1 to 30 µm, more preferably from 5 to 25 µm, better still from 5 to 20 µm and even better still from 5 to 15 µm.

According to an advantageous embodiment, the hydrophobic silica aerogel particles used in the present invention have a specific surface area per unit mass (S_{M}) ranging from 600 to 800 m²/g and a size expressed as the volume-average diameter (D[0.5]) ranging from 5 to 20 µm and even better still from 5 to 15 µm.

The specific surface area per unit mass may be determined by the nitrogen absorption method, known as the BET (Brunauer-Emmett-Teller) method, described in The Journal of the American Chemical Society, vol. 60, page 309, February 1938 and corresponding to international standard ISO 5794/1 (annex D). The BET specific surface corresponds to the total specific surface of the particles under consideration.

The sizes of the silica aerogel particles can be measured by static light scattering using a commercial particle size analyser of MasterSizer 2000 type from Malvern. The data are processed on the basis of the Mie scattering theory. This theory, which is exact for isotropic particles, makes it possible to determine, in the case of non-spherical particles, an "effective" particle diameter. This theory is in particular described in the publication by Van de Hulst, H.C., Light Scattering by Small Particles, Chapters 9 and 10, Wiley, New York, 1957.

According to a preferred embodiment, the hydrophobic silica aerogel particles used in the present invention have a specific surface area per unit of volume S_{V} ranging from 5 to 60 m²/cm³, preferably from 10 to 50 m²/cm³ and better still from 15 to 40 m²/cm³.

The aerogels used according to the present invention are hydrophobic silica aerogels, preferably aerogels of silyl silica (INCI name: silica silylate).

As regards the preparation of hydrophobic silica aerogel particles modified at the surface by silylation, reference may be made to the document US 7 470 725.

Use will preferably be made of hydrophobic silica aerogel particles modified at the surface by trimethylsilyl groups.

As hydrophobic silica aerogels that may be used in the invention, examples that may be mentioned include the aerogel sold under the name VM-2260 (INCI name: Silica silylate), by the company Dow Corning, the particles of which have an average size of about 1000 microns and a specific surface area per unit mass ranging from 600 to 800 m²/g.

Mention may also be made of the aerogels sold by Cabot under the references Aerogel TLD 201, Aerogel OGD 201, Aerogel TLD 203, Enova^{®} Aerogel MT 1100 and Enova Aerogel MT 1200.

Use will preferably be made of the aerogel sold under the name VM-2270 (INCI name: Silica silylate) by the company Dow Corning, the particles of which have an average size ranging from 5-15 microns and a specific surface area per unit mass ranging from 600 to 800 m2/g.

Preferably, the mineral thickeners are chosen from organophilic clays, in particular modified hectorites; hydrophobic treated fumed silica; hydrophobic silica aerogels, or mixtures thereof, and more specifically still at least one organophilic modified clay or at least one hydrophobic modified silica, in particular an organophilic modified clay.

More particularly, if the composition contains it (them), the content of mineral thickener(s) represents from 0.2% to 2.5% by weight, expressed as active material, and preferably from 0.5% to 2% by weight, relative to the weight of the composition.

The example hereinafter is given as a non-limiting illustration of the field of the invention.

### EXAMPLE

### 1. Composition

The composition of which the ingredients are collated in the table below is prepared; the amounts are expressed in weight amount of starting material:

| Phase | Ingredient | Composition 1 | Composition 2 |
|---|---|---|---|
| A | Cetyl PEG/PPG-10/1 Dimethicone (ABIL EM 90 sold by Evonik Goldschmidt) | 3 | 3 |
| | Polyglyceryl-4 Isostearate (Isolan GI34 sold by | 1 | 1 |
| | Evonik Goldschmidt) | | |
| | PEG/PPG-18/18 Dimethicone (KF6050L sold by Shin Etsu) | 0.3 | 0.3 |
| | Isododecane | 2.7 | 2.7 |
| | Dodecamethylpentasiloxane (Xiameter PMX-200 Silicone Fluid 2CS; sold by Dow Corning) | 11.5 | 10.5 |
| | Trimethyl pentaphenyl trisiloxane (Dow Corning PH-1555 HRI Cosmetic Fluid sold by Dow Corning) | 2.2 | 2.2 |
| | 2-Octyldodecanol | 10 | 15 |
| | Acrylates/polytrimethylsiloxy methacrylate copolymer (Dow Corning FA 4002 ID silicone acrylate sold by Dow Corning, as a 40% by weight mixture in isododecane) | 10 | 10 |
| B | Disteardimonium hectorite | 1 | 1 |
| | Propylene carbonate | 0.3 | 0.3 |
| | Dodecamethylpentasiloxane (Xiameter PMX-200 Silicone Fluid 2CS; sold by Dow Corning) | 3.7 | 3.7 |
| C | Red 7 | 1.5 | 0.9 |
| | Red 28 Lake | 1.3 | 0.6 |
| | Anatase titanium oxide coated with aluminium stearoyl glutamate (97/3) | 1.8 | 0.9 |
| | black iron oxide coated with aluminium stearoyl glutamate (3%) | 1.4 | 0.7 |
| | Yellow 6 Lake | 2.1 | 1 |
| D | Water | 34.5 | 34.5 |
| | Butylene glycol | 6 | 6 |
| | Magnesium sulfate | 0.7 | 0.7 |
| | Ethanol | 5 | 5 |

### 2. Preparation

First of all, the pigments are mixed into a part of the dodecamethylpentasiloxane of phase A (in an Exakt three-roll machine).

Separately, the aqueous phase D is prepared using a magnetic bar.

Separately, the ingredients of phase B are mixed by means of a Rayneri mixer.

The ingredients of phase A, the pigments/ dodecamethylcyclopentasiloxane mixture previously obtained are then mixed, with Moritz stirring, for 20 minutes, at ambient temperature.

Once the mixture A is smooth and homogeneous and phase B is homogeneous, the emulsion is prepared at ambient temperature by pouring the aqueous phase D onto phase A, by means of a Moritz stirrer.

Once the mixing has been carried out, the stirring is continued until a homogeneous product is obtained and the whole mixture is packaged in a container fitted with a dip stirrer corresponding to Figure 1.

The compositions obtained are homogeneous and stable. There is no release (for instance no apparition of a continuous or discontinuous layer of oil) or sedimentation, after treatment in the centrifuge (900 g for 1 hour).

After one week, they do not undergo any phase separation at room temperature or in an oven at 45°C (more particularly no release (i.e. no apparition of a continuous or discontinuous layer of oil), no sedimentation, no apparition of cracks in the composition).

The viscosity of composition 1 is 1.4 Pa.s and the viscosity of composition 2 is 1.3 Pa.s (Rheomat 180 viscosimeter, spindle 3, 25°C; 10 minutes, 200rpm).

The device makes it possible to apply a very thin film to the lips, which is comfortable and which does not stick.

The composition applied does not migrate after one hour and the film wear is satisfactory. The film also has good wear.

It should be noted that, if a conventional applicator is used for lipgloss (for example a container fitted with a flocked flexible applicator of reference 14030, Geka GmbH), a thicker, stickier deposit is obtained, the resistance of which to migration and to transfer is not as good as when using the applicator according to the invention.

### Protocol for measuring the film thickness:

This protocol is an *in vitro* measurement.
- A square of Bioskin^{®} synthetic skin of 3 cm / 4 cm is prepared.
- The skin square obtained is weighed.
- The composition is applied by means of the device so as to obtain an even deposit covering the entire surface of the skin square.
- The skin square thus made-up is weighed.

### Thickness of the film:

Thickness (cm) = volume of composition applied (g): density of the composition (g/cm³).

The density of the composition is 1.

The average thickness is given with three separate measurements.

## Claims

1. Cosmetic composition which is in the form of a liquid emulsion comprising:
∘ water;
∘ at least one film-forming agent chosen from vinyl polymers comprising at least one carbosiloxane dendrimer-based unit chosen from polymers of which the INCI name is Acrylates / Polytrimethylsiloxy Methacrylate Copolymer
∘ from 5% to 15% by weight, relative to the weight of the composition, of at least one polar non-volatile hydrocarbon-based oil;
∘ from 1% to 8% by weight, relative to the weight of the composition, of at least one non-volatile phenyl silicone oil, preferably without dimethicone fragment.

2. Composition according to the preceding claim, **characterized in that** the non-volatile polar hydrocarbon-based oil(s) is (are) chosen from C₁₀-C₂₆ alcohols, optionally hydroxylated monoesters, diesters or triesters of a C₂-C₈ monocarboxylic or polycarboxylic acid and of a C₂-C₈ alcohol, optionally hydroxylated monoesters, diesters or triesters of a C₂-C₈ monocarboxylic or polycarboxylic acid and of a C₂-C₈ alcohol, esters of a C₂-C₈ polyol and of one or more C₂-C₈ carboxylic acids, ester oils, in particular having between 18 and 70 carbon atoms, vinylpyrrolidone/1-hexadecene copolymers, C₁₂-C₂₆ fatty acids, dialkyl carbonates, the 2 alkyl chains possibly being identical or different, and mixtures thereof, and preferably from C₁₀-C₂₆ alcohols; preferably C₂₀-C₂₆ alcohols.

3. Composition according to any one of claims 1 or 2, **characterized in that** the content of non-volatile polar hydrocarbon-based oil(s) is between 6% and 12% by weight relative to the weight of the composition.

4. Composition according to any one of claims 1 to 3, **characterized in that** the content of non-volatile phenyl silicone oil(s) preferably not having a dimethicone fragment ranges from 1.5% to 5% by weight, relative to the weight of the composition.

5. Composition according to any one of claims 1 to 4, **characterized in that** the content of non-volatile oils represents from 6% to 20% by weight and preferably from 6% to 15% by weight relative to the weight of the composition.

6. Composition according to any one of claims 1 to 5, **characterized in that** the content of film-forming agent(s) represents from 0.5% to 30% by weight of active material and preferably from 1% to 20% by weight, relative to the weight of the composition.

7. Composition according to any one of claims 1 to 6, **characterized in that** the composition comprises at least one volatile, preferably hydrocarbon-based or silicone, oil.

8. Composition according to any one of claims 1 to 7, **characterized in that** the content of volatile oil(s) represents from 5% to 45% by weight and preferably from 10% to 30% by weight, relative to the weight of the composition.

9. Composition according to any one of claims 1 to 8, **characterized in that** the water content represents from 20% to 60% by weight relative to the weight of the composition.

10. Composition according to any one of claims 1 to 9, **characterized in that** the composition is in the form of an inverse (water-in-oil) emulsion.

11. Composition according to any one of claims 1 to 10, **characterized in that** the composition comprises at least one surfactant, preferably chosen from non-ionic surfactants and silicone surfactants, or mixtures thereof.

12. Composition according to any one of claims 1 to 11, **characterized in that** the composition has a viscosity at 25°C. of between 0.005 and 12 Pa.s, preferably between 0.01 and 10 Pa.s and even more advantageously between 0.05 and 8 Pa.s and more particularly between 0.1 to 6 Pa.s.

13. Process for making up and/or caring for the lips, in which the composition according to any one of the claims 1 to 12 is applied to the lips.

## Patentansprüche

1. Kosmetische Zusammensetzung, die in Form einer flüssigen Emulsion vorliegt, umfassend:
o Wasser;
o mindestens einen Filmbildner, ausgewählt aus Vinylpolymeren mit mindestens einer auf Carbosiloxandendrimer basierenden Einheit, ausgewählt aus Polymeren, deren INCI-Name Acrylates / Polytrimethylsiloxy Methacrylate Copolymer lautet,
o 5 bis 15 Gew.-%, bezogen auf das Gewicht der Zusammensetzung, mindestens eines polaren nichtflüchtigen kohlenwasserstoffbasierten Öls;
o 1 bis 8 Gew.-%, bezogen auf das Gewicht der Zusammensetzung, mindestens eines nichtflüchtigen Phenylsilikonöls, vorzugsweise ohne Dimethicon-Fragment.

2. Zusammensetzung nach dem vorhergehenden Anspruch, **dadurch gekennzeichnet, dass** das nichtflüchtige polare kohlenwasserstoffbasierte Öl bzw. die nichtflüchtigen polaren kohlenwasserstoffbasierten Öle aus C₁₀-C₂₆-Alkoholen, gegebenenfalls hydroxylierten Monoestern, Diestern oder Triestern einer C₂-C₈-Mono- oder Polycarbonsäure und einem C₂-C₈-Alkohol, gegebenenfalls hydroxylierten Monoestern, Diestern oder Triestern einer C₂-C₈-Mono- oder Polycarbonsäure und einem C₂-C₈-Alkohol, Estern eines C₂-C₈-Polyols und einer oder mehrerer C₂-C₈-Carbonsäuren, Esterölen, insbesondere mit zwischen 18 und 70 Kohlenstoffatomen, Vinylpyrrolidon/1-Hexadecen-Copolymeren, C₁₂-C₂₆-Fettsäuren, Dialkylcarbonaten, wobei die 2 Alkylketten gleich oder verschieden sein können, und Mischungen davon und vorzugsweise aus C₁₀-C₂₆-Alkoholen; vorzugsweise C₂₀-C₂₆-Alkoholen; ausgewählt ist bzw. sind.

3. Zusammensetzung nach einem der Ansprüche 1 oder 2, **dadurch gekennzeichnet, dass** der Gehalt an nichtflüchtigem polarem kohlenwasserstoffbasiertem Öl bzw. nichtflüchtigen polaren kohlenwasserstoffbasierten Ölen zwischen 6 und 12 Gew.-%, bezogen auf das Gewicht der Zusammensetzung, liegt.

4. Zusammensetzung nach einem der Ansprüche 1 bis 3, **dadurch gekennzeichnet, dass** der Gehalt an nichtflüchtigem Phenylsilikonöl bzw. nichtflüchtigen Phenylsilikonölen, das bzw. die vorzugsweise kein Dimethicon-Fragment aufweist bzw. aufweisen, im Bereich von 1,5 bis 5 Gew.-%, bezogen auf das Gewicht der Zusammensetzung, liegt.

5. Zusammensetzung nach einem der Ansprüche 1 bis 4, **dadurch gekennzeichnet, dass** der Gehalt an nichtflüchtigen Ölen 6 bis 20 Gew.-% und vorzugsweise 6 bis 15 Gew.-%, bezogen auf das Gewicht der Zusammensetzung, ausmacht.

6. Zusammensetzung nach einem der Ansprüche 1 bis 5, **dadurch gekennzeichnet, dass** der Gehalt an Filmbildner(n) 0,5 bis 30 Gew.-% Aktivsubstanz und vorzugsweise 1 bis 20 Gew.-%, bezogen auf das Gewicht der Zusammensetzung, beträgt.

7. Zusammensetzung nach einem der Ansprüche 1 bis 6, **dadurch gekennzeichnet, dass** die Zusammensetzung mindestens ein flüchtiges Öl, vorzugsweise ein kohlenwasserstoffbasiertes Öl oder Silikonöl, umfasst.

8. Zusammensetzung nach einem der Ansprüche 1 bis 7, **dadurch gekennzeichnet, dass** der Gehalt an flüchtigem Öl bzw. flüchtigen Ölen 5 bis 45 Gew.-% und vorzugsweise 10 bis 30 Gew.-%, bezogen auf das Gewicht der Zusammensetzung, ausmacht.

9. Zusammensetzung nach einem der Ansprüche 1 bis 8, **dadurch gekennzeichnet, dass** der Wassergehalt 20 bis 60 Gew.-%, bezogen auf das Gewicht der Zusammensetzung, ausmacht.

10. Zusammensetzung nach einem der Ansprüche 1 bis 9, **dadurch gekennzeichnet, dass** die Zusammensetzung in Form einer inversen Emulsion (Wasser-in-Öl-Emulsion) vorliegt.

11. Zusammensetzung nach einem der Ansprüche 1 bis 10, **dadurch gekennzeichnet, dass** die Zusammensetzung mindestens ein Tensid umfasst, das vorzugsweise aus nichtionischen Tensiden und Silikontensiden oder Mischungen davon ausgewählt ist.

12. Zusammensetzung nach einem der Ansprüche 1 bis 11, **dadurch gekennzeichnet, dass** die Zusammensetzung eine Viskosität bei 25 °C zwischen 0,005 und 12 Pa.s, vorzugsweise zwischen 0,01 und 10 Pa.s und noch vorteilhafter zwischen 0,05 und 8 Pa.s und spezieller zwischen 0,1 bis 6 Pa.s aufweist.

13. Verfahren zum Schminken und/oder Pflegen für die Lippen, bei dem man die Zusammensetzung nach einem der Ansprüche 1 bis 12 auf die Lippen aufbringt.

## Revendications

1. Composition cosmétique qui est sous la forme d'une émulsion liquide comprenant :
o de l'eau ;
o au moins un agent filmogène choisi parmi des polymères vinyliques comprenant au moins un motif à base de dendrimère de carbosiloxane choisi parmi des polymères dont le nom INCI est Acrylates / Polytrimethylsiloxy Methacrylate Copolymer
o de 5 % à 15 % en poids, par rapport au poids de la composition, d'au moins une huile hydrocarbonée non volatile polaire ;
o de 1 % à 8 % en poids, par rapport au poids de la composition, d'au moins une huile de phénylsilicone non volatile, préférablement sans fragment diméthicone.

2. Composition selon la revendication précédente, **caractérisée en ce que** l'huile ou les huiles hydrocarbonées polaires non volatiles sont choisies parmi des alcools en C10-C26, des monoesters, diesters ou triesters éventuellement hydroxylés d'un acide monocarboxylique ou polycarboxylique en C2-C8 et d'un alcool en C2-C8, des monoesters, diesters ou triesters éventuellement hydroxylés d'un acide monocarboxylique ou polycarboxylique en C2-C8 et d'un alcool en C2-C8, des esters d'un polyol en C2-C8 et d'un ou plusieurs acides carboxyliques en C2-C8 des huiles d'ester, en particulier ayant entre 18 et 70 atomes de carbone, des copolymères de vinylpyrrolidone/1-hexadécène, des acides gras en C12-C26, des carbonates de dialkyle, les 2 chaînes alkyle étant possiblement identiques ou différentes et des mélanges correspondants et préférablement parmi des alcools en C10-C26 ; préférablement des alcools en C20-C26.

3. Composition selon l'une quelconque des revendications 1 et 2, **caractérisée en ce que** la teneur en huile(s) hydrocarbonée(s) polaire(s) non volatile(s) est comprise entre 6 % et 12 % en poids par rapport au poids de la composition.

4. Composition selon l'une quelconque des revendications 1 à 3, **caractérisée en ce que** la teneur en huile(s) de phénylsilicone non volatile(s) préférablement n'ayant pas un fragment diméthicone se situe dans la plage de 1,5 % à 5 % en poids, par rapport au poids de la composition.

5. Composition selon l'une quelconque des revendications 1 à 4, **caractérisée en ce que** la teneur en huiles non volatiles représente de 6 % à 20 % en poids et préférablement de 6 % à 15 % en poids par rapport au poids de la composition.

6. Composition selon l'une quelconque des revendications 1 à 5, **caractérisée en ce que** la teneur en agent(s) filmogène(s) représente de 0,5 % à 30 % en poids de matériau actif et préférablement de 1 % et 20 % en poids par rapport au poids de la composition.

7. Composition selon l'une quelconque des revendications 1 à 6, **caractérisée en ce que** la composition comprend au moins une huile volatile, préférablement de silicone ou hydrocarbonée.

8. Composition selon l'une quelconque des revendications 1 à 7, **caractérisée en ce que** la teneur en huile (s) volatile (s) représente de 5 % à 45 % en poids et préférablement de 10 % à 30 % en poids par rapport au poids de la composition.

9. Composition selon l'une quelconque des revendications 1 à 8, **caractérisée en ce que** la teneur en eau représente de 20 % à 60 % en poids par rapport au poids de la composition.

10. Composition selon l'une quelconque des revendications 1 à 9, **caractérisée en ce que** la composition est sous la forme d'une émulsion inverse (eau-dans-huile).

11. Composition selon l'une quelconque des revendications 1 à 10, **caractérisée en ce que** la composition comprend au moins un tensioactif, préférablement choisi parmi des tensioactifs non ioniques et des tensioactifs de type silicone, ou des mélanges correspondants.

12. Composition selon l'une quelconque des revendications 1 à 11, **caractérisée en ce que** la composition possède une viscosité à 25 °C comprise entre 0,005 et 12 Pa.s, préférablement entre 0,01 et 10 Pa.s et encore plus avantageusement entre 0,05 et 8 Pa.s et plus particulièrement entre 0,1 et 6 Pa.s.

13. Procédé pour le maquillage et/ou l'entretien pour les lèvres, dans lequel la composition selon l'une quelconque des revendications 1 à 12 est appliquée sur les lèvres.
